# EUROPEAN PATENT SPECIFICATION

(11) **EP 2 976 060 B1**
(45) Date of publication and mention of the grant of the patent: **26.10.2016**
(21) Application number: 14713795.4
(22) Date of filing: 21.03.2014
(51) Int. Cl.: A61K 8/73, A61Q 19/08, A61Q 7/00

(54) **EXOPOLYSACCHARIDE FOR THE TREATMENT AND/OR CARE OF THE SKIN, MUCOUS MEMBRANES AND/OR NAILS**
EXOPOLYSACCHARIDE ZUR BEHANDLUNG UND/ODER ZUM SCHUTZ DER HAUT, SCHLEIMHÄUTE UND/ODER NÄGEL
EXOPOLYSACCHARIDES POUR LE TRAITEMENT ET/OU LE SOIN DE LA PEAU, DES MUQUEUSES ET/OU DES ONGLES

(30) Priority: 22.03.2013 EP 13382107
(43) Date of publication of application: 27.01.2016
(73) Proprietor: Lipotec, S.A., 08850 Gavà Barcelona (ES); Polymaris Biotechnology, 29600 Morlaix (FR)
(72) Inventor: DELGADO GONZALEZ, Raquel, E-08550 Gavà (ES); SOLEY ASTALS, Albert, E-08550 Gavà (ES); COURTOIS, Anthony, F-29600 Morlaix (FR); THOLLAS, Bertrand, F-29600 Morlaix (FR)
(74) Representative: Bradley, Josephine Mary
(86) International application number: PCT/EP2014/055775
(87) International publication number: WO 2014/147255

(56) References cited:
- WO-A2-2012/072245
- FR-A1- 2 894 146
- FR-A1- 2 894 147

## Description

### FIELD OF THE INVENTION

This invention relates to an exopolysaccharide (EPS), which inhibits neuronal exocytosis and stimulates the fibroblast proliferation. Said exopolysaccharide is excreted by the strain of the *Vibrio sp* species with deposit number CNCM I-4239. This invention also relates to the use of this exopolysaccharide in cosmetic or dermopharmaceutical compositions for the treatment and/or care of the skin, mucous membranes, hair and/or nails.

### BACKGROUND

One of the strategies in the cosmetic industry for the prevention and reduction of the wrinkles, in particular, expression wrinkles, is the administration of compounds which block the muscle contraction by inhibition of neuronal exocytosis in that area. The principle muscles involved in the appearance of expression lines are those surrounding the eyes and eyelashes, those on the forehead, the lip, mouth, cheek and neck muscles. These muscles are found in the subcutaneous connective frontal part of the face, from where they rise towards the skin and insert themselves in the deepest part of the dermal stratum. Their contraction can lead to raising, depressing, constricting or dilatory movements of the skin. The early appearance of wrinkles is the most characteristic sign of age and aging of the skin.

The use of the toxin *Clostridium botulinum* (marketed as Botox^{®} by Allergan) injected into the muscle to reduce muscle contraction and to treat associated diseases such as dystonia and/or pain has spread since the decade of 1990. Neurotoxin injections have also been used to treat and/or care for the skin with the aim of reducing, delaying or preventing the signs of aging and/or photoaging and in particular to relax the facial expression and reduce the formation of wrinkles or minimize their appearance. Its action mechanism is based on blocking acetyl choline release in the presynaptic terminal of the axon in the neuromuscular junction, thus avoiding nerve transmission and muscle contraction. The toxin binds to receptors in the presynaptic membrane, is internalized and goes through the cytoplasm. Its activity is responsible for breaking the trimolecular SNARE complex of synaptobrevin, SNAP-25 and syntaxin, what avoids the binding of synaptic vesicles to plasmalemma and the release of acetylcholine to the synaptic cleft. The controlled administration of the botulinum toxin has been used for the treatment of a wide range of conditions, disorders and diseases, such as perspiration and hyperhidrosis (US 6974578 B2 and US 6683049 B2), different disorders and diseases of the skin such as calluses, warts, ulcers and lesions on the skin (US 8048423 B2, US 2011/206731), psoriasis and dermatitis (US 5670484 A), vascular hyperreactivity and rosacea (WO 2010/114828), acne (WO 03/011333), hair growth and maintenance (US 6299893 B1), facial wrinkles (US 7255865 B2), ptosis of the eyebrows and forehead (US 2011/280978) or drooping mouth corners (US 6358917 B1) and different types of pain and inflammation (US 2010/266638, US 7811586 B2, US 7704524 B2, US 7704511 B2, US 7468189 B2, US 7255866 B2, US 7091176 B2, US 6887476 B2, US 6869610 B2, US 6838434 B2, US 6641820 B2, US 6623742 B2, US 6565870 B1, US 6500436 B1, US 6458365 B1, US 6423319 B1, US 6113915 A, US 5714468 A and US 6063768 B2) among others.

However, the toxicity inherent in botulinum toxin causes its administration, in a wide range of doses, to result in undesired secondary effects, such as immunogenic responses, cephalalgias, nausea, paralysis or muscle weakness, respiratory failure, and in more extreme cases even the death of the subject treated *[*FDA News, February 8, 2008, "FDA Notifies Public of Adverse Reactions Linked to Botox Use*";* Coté, T.R. et al. "Botulinum toxin type A injections: Adverse events reported to the US Food and Drug Administration in therapeutic and cosmetic cases" J. Amer. Acad. Derm. 2005, 53 (3), 407-415*].* These severe secondary effects, together with the high cost of the treatment, seriously limits the application of botulinum toxin with therapeutic or cosmetic purposes, being relegated to chronic applications and/or diseases for which there is no suitable treatment. There is, therefore, a pressing need to develop molecules which imitate the paralyzing effects of botulinum toxins but which are equipped with much simpler and more stable molecular structures that do not induce immune reactions, and whose cost of production is affordable. Molecules of a peptide nature comply with these properties.

Wrinkles are related with multiple biological changues in the dermis. In the dermis, there is a decrease in the number or dermic fibroblasts which produce the different components of the extracellular matrix responsible for the firmness and elasticity of the skin and the restoration of the mechanical properties of the skin.

During the wound healing process there are several other processes which assist in the regeneration of the skin and which can be also useful in anti-wrinkle treatments. In the healing process, there is an increase in the cell proliferation and migration *[*Grose et al., "A crucial role of β1 integrins for keratinocyte migration in vitro and during cutaneous wound repair", Development., 2002, 2303-2315*]* which would help to the reepithelialization revertint the diminution of the thickness of the epidermis related with the age *[*Varani et al., "Vitamin A Antagonizes Decreased Cell Growth and Elevated Collagen-Degrading Matrix Metalloproteinases and Stimulates Collagen Accumulation in Naturally Aged Human Skin", J. Invest. Dermatol., 2000, 114, 480-486*],* and which is more specifically observed in the areas where wrinkles are more frequent (around the eyes, forehead, nasogenian area, among other areas). Afterwards, during the healing, there is an increase in the fibroblast proliferation which are responsible for carrying out the synthesis of extracellular matrix which also assist to the diminution of wrinkles.

The existence of exopolysaccharides has been known since the 1970s, they are produced by species of bacteria which live in ecosystems known for their extreme conditions. The production of exopolysaccharides by the bacteria which live in these ecosystems is principally related to functions of survival (Raguénès et al. JAppl Microbiol., 1997 Apr, 82(4):422-30).

In the cosmetic field, the polysaccharides have been widely used, mainly as gelling and/or texturizing agents that are added to formulations of final products. It is known in the prior art that certain exopolysaccharides have been widely used for cosmetic or/and dermopharmaceutical purposes, such as the exopolysaccharide produced by a bacterial strain of *Pseudomonas* genus described in the patent EP0534855B1, which is used as a thickening, gelling and/or texturising agent. The patent application FR2871476A1 describes the strain GY785 of hydrotermal origin of the genus Alteromonas that produces an exopolysaccharide which has shown utility as a healing agent; EP0987010B1 patent describes an exopolysaccharide produced by a mesophilic bacteria of hydrothermal origin which improves the defense system of the skin and the patent application US2010/009931 describes that the exopolysaccharide produced by a microalga strain of the genus *Porphyridium* as tensor agent, which also improves the firmness, elasticity and tonicity of the skin. Similarly, the patent application US2009/069213A1 discloses a microalga strain *Porphyridium sp.* producing a polysaccharide showing anti-wrinkle and moisturizing properties. Furthermore, the patent US6344346B1 describes cosmetic compositions with moisturizing properties caused by a natural polysaccharide excreted from a bacterial strain of *Rhizobium* genus.

Another exopolysaccharide which has shown to have many advantageous properties for the skin is the exopolysaccharide described in the application WO2009/127057, exopolysaccharide produced by strains of the bacterial species *Staphylococcus epidermidis* or *Staphylococcus aureus.* After applying a cosmetic composition of this exopolysaccharide, the hydration and morphology of the stratum corneum is improved, and the peeling of the skin

Furthermore, it should be mentioned that the patent application JP2003-313131 describes a sulfated polysaccharide produced by a strain of *Alteromonas sp.* SN-1009 (FERM BP-5747) with anti-wrinkle properties.

Finally, the application WO 2012/072245 describes an exopolysaccharide produced by an strain of *Pseudoalteromonas sp.* with moisturizing properties, and the patent application P2012-30432 discloses an exopolysaccharide produced by a strain of *Vibrio sp.* which stimulates the production of hyaluronic acid in skin cells.

Surprisingly the applicant of this invention has found activity in a new exopolysaccharide excreted by the no-hydrothermal bacterial strain *Vibrio sp.* under deposit number CNCM I-4239 according to the Budapest Treaty which inhibits neuronal exocytosis and prevents and/or reduce skin wrinkles and additionally it stimulates the fibroblast proliferation and it increases the firmness of the skin.

### DETAILED DESCRIPTION OF THE INVENTION

This invention relates to the cosmetic and/or dermopharmaceutical use of the exopolysaccharide excreted by the bacterial strain of the species *Vibrio sp.* with deposit number CNCM I-4239. Surprisingly the inventors of this invention have found that the aforementioned exopolysaccharide inhibits neuronal exocytosis and also stimulates fibroblast proliferation. Particularly, the inhibition of neuronal exocytosis reduces and/or prevents skin wrinkles, and the stimulation of fibroblast proliferation increase the firmness of the skin.

### Definitions

In order to facilitate the comprehension of this invention, the meanings of some terms and expressions as used in the context of the invention are included.

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes and/or adipocytes among others. In the context of this invention, the term "skin" includes the scalp.

The term "treatment", as used in the context of this specification when it is not accompanied by the qualifications "cosmetic, non-therapeutic", means the administration of a compound according to the invention to alleviate or eliminate a disease or disorder or reduce or eliminate one or more symptoms associated with this disease or disorder. The term "treatment" also covers the ability to alleviate or eliminate the physiological consequences of the disease or disorder.

When the term "treatment" is accompanied by the qualifications "cosmetic, non-therapeutic" they refer to the application of the compound to the skin, hair and/or mucous membranes in particular with the aim of improving the cosmetic qualities of the skin, hair and/or mucous membranes such as and not restricted to, their level of hydration, elasticity, firmness, shine, tone or texture, among others. The term "care" in this invention refers to the maintenance of the qualities of the skin, hair and/or mucous membranes. These qualities are subject to improvement and maintained through a cosmetic treatment and/or care of the skin, hair and/or mucous membranes both in healthy subjects as well as those which present diseases and/or disorders of the skin and/or mucous membranes, such as and not restricted to, ulcers and lesions on the skin, psoriasis, dermatitis, acne or rosacea, among others.

The term "prevention", as used in this invention, refers to the ability of a compound of the invention to prevent, delay or hinder the appearance or development of a disease or disorder before its appearance.

In the context of this invention, the term "aging" refers to the changes experienced by the skin with age (chronoaging) or through exposure to the sun (photoaging) or to environmental agents such as tobacco smoke, extreme climatic conditions of cold, heat, or wind, chemical contaminants or pollutants, and includes all the external visible and/or perceptible changes through touch, such as and not restricted to, the development of discontinuities on the skin such as wrinkles, fine lines, furrows, irregularities or roughness, increase in the size of pores, loss of elasticity, loss of firmness, loss of smoothness, loss of the capacity to recover from deformation, sagging of the skin such as sagging cheeks, the appearance of bags under the eyes or the appearance of a double chin, among others, changes to the color of the skin such as marks, reddening, bags under the eyes or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and present the same physical characteristics as aging, such as and not restricted to, flaccidity, sagging, changes to the color or irregularities in the pigmentation, abnormal and/or excessive keratinization.

The strain which produces the exopolysaccharide of this invention was deposited in accordance with the Budapest Treaty, on September 4, 2009, in the "Collection Nationale de Culture de Microorganismes" [National Microorganism Culture Collection] (CNCM), Pasteur Institute, 28 rue du Docteur Roux, 75724 Paris, France, under code CNCM I-4239.

Thus, a first aspect of this invention relates to the exopolysaccharide of the strain of the species *Vibrio sp.* with deposit number CNCM I-4239 for its use in the treatment of the skin, mucous membranes, hair and/or nails. In particular, the treatment relates to the treatment and/or prevention of the pain, inflammation, itching or hyperhidrosis, or re-epithelizing and/or healing treatment of the skin and/or mucous membranes.

In a preferred embodiment, the pain is selected from pain associated with conditions, diseases and/or disorders, for example and not restricted to, touch sensitivity, sensitivity to cold, sensitivity to heat, cutaneous irritation, post-hair removal cutaneous irritation, post-shaving cutaneous irritation, psoriasis, sensitive skin, dermatitis, atopic dermatitis, contact dermatitis, diaper dermatitis, seborrheic dermatitis, eczema, lichen planus, burns, sunburn, arthritis, rheumatoid arthritis, osteoarthritis, psoriatic arthritis, hypersensitivity, cutaneous pain or irritation after surgery, after intense pulsed light treatment (IPL), after treatment with monochromatic pulsed light (laser), after a treatment with chemical desquamating agents or after overexposure to external aggressive agents, among others.

In another preferred embodiment, the inflammation is selected from, for example and not restricted to, psoriasis, sensitive skin, dermatitis, atopic dermatitis, contact dermatitis, diaper dermatitis, seborrheic dermatitis, eczema, rosacea, acne, hyperproliferative skin disease, burns, sunburn, paronychia, cutaneous inflammation after surgery, after intense pulsed light treatment (IPL), after treatment with monochromatic pulsed light (laser), after a treatment with chemical desquamating agents or after overexposure to external aggressive agents, inflammation of the mucous membrane of the vagina, inflammation of the oral mucous membranes, gingivitis, periodontitis, rhinitis, allergic rhinitis, among others.

In another preferred embodiment, the itching is selected from itching associated with conditions, diseases and/or disorders, for example and not restricted to, dermatitis, atopic dermatitis, contact dermatitis, diaper dermatitis, dermatitis herpetiformis, photodermatosis, photosensitivity, pregnancy related dermatitis, menopause related dermatitis, eczema, sensitive skin, psoriasis, chickenpox, herpes, herpes zoster, Netherton's syndrome, peeling skin syndrome, lichen planus, acne, dandruff, seborrhea, seborrheic dermatitis, alopecia, athlete's foot, candidiasis, hemorrhoids, vaginal itching, perianal itching, anogenital itching, sunburn, hives, pruritic otitis, itchy eyes, senile pruritus, aquagenic pruritus, prurigo nodularis, prurigo planus, pityriasis rosea, xerosis and dry skin, allergic reactions, allergies to medicines, food allergies, allergies to chemical products, exposure to poisonous plants and exposure to insect bites, among others.

In another aspect, the present invention relates to the use of the exopolysaccharide of the strain of the species *Vibrio sp.* with deposit number CNCM I-4239 for the cosmetic, non-therapeutic treatment and/or care of the skin, mucous membranes, hair and/or nails, in particular for the treatment and/or prevention of skin aging, for the treatment and/or prevention of skin wrinkles, preferably expression wrinkles, for the treatment and/or prevention of loss of skin firmness, for the treatment and/or prevention of perspiration, treatment and/or care of skin disorders selected from the group formed by warts, calluses, treatment stimulating hair growth and/or prevention of hair loss.

Preferably, the treatment, the cosmetic, non-therapeutic treatment and/or care of the skin, mucous membranes, hair and/or nails inhibits the neuronal exocytosis.

Preferably, the treatment, the cosmetic, non-therapeutic treatment and/or care of the skin, mucous membranes, hair and/or nails stimulates the fibroblast proliferation.

Preferably, the treatment and/or prevention of hyperhidrosis or perspiration, is a treatment and/or prevention of axillary, facial, genital, palmar or plantar hyperhidrosis or perspiration

.In another particular embodiment, the treatment and/or care of the skin, mucous membranes, hair and/or nails is carried out by topical or transdermal application.

In another particular embodiment, the exopolysaccharide can be obtained through fermentation of the strain of the species *Vibrio sp.* with deposit number CNCM I-4239 in a suitable culture medium, conventionally stirred and aerated. Fermentation to produce the exopolysaccharide of this invention can be carried out in a medium stirred and aerated at a temperature between 15°C and 37°C, preferably at 25°C, the medium having a pH between 6.5 and 9, preferably around 7.5, adjusting it if necessary during fermentation. The duration of the fermentation is between 30 to 120 hours, preferably between 48 and 96 hours.

In a particular embodiment, in the fermentation of the strain of the species *Vibrio sp.* with deposit number CNCM I-4239 of the invention exogenous sugars, such as and not restricted to, galactose, glucose, mannose, amygdalin, cellobiose, maltose, starch, glycogen, lactose, mixtures thereof and/or extracts containing mixtures of these sugars can be used as a source of carbon. In particular, an exogenous supply of glucose of 2 to 40 g/L, and preferably from 15 to 25 g/L is provided. Methods of incorporation of sugars to produce different polysaccharides are described in the prior art, such as and not restricted to in documents: WO 98/38327, *"*Vibrio diabolicus sp. nov., a new polysaccharide-secreting organism isolated from a deep-sea hydrothermal vent polychaete annelid, Alvinella pompejana"; Raguénès et al., Int. J. Syst. Bact., (1997), 47, 989-995 and *"*Structure of the exopolysaccharide of Vibrio diabolicus isolated from a deep-sea hydrothermal vent"; Rougeaux et al., Carbohydrate.. Res., (1999), 322:40-45*.*

In another particular embodiment, mineral salts are also provided to the fermentation culture of the strain of the species *Vibrio sp.* with deposit number CNCM I-4239 and they are selected from among salts which provide the ions Na⁺, K⁺, NH₄⁺, Ca²⁺, Mg²⁺, PO₄³⁻, SO₄²⁻, Cl⁻, CO₃²⁻, or trace elements such as Cu, Mn, Fe and Zn.

In another particular embodiment, the method of isolation and purification of the exopolysaccharide is carried out by the methods known by the person skilled in the art such as, centrifugation, filtration, ultrafiltration and dialysis. Preferably ultrafiltration and dialysis are carried out with a polyethersulfone membrane which retains molecules of a molecular weight greater than 100,000 Da.

In another particular embodiment this invention relates to the native exopolysaccharide and any chemical modification known by the person skilled in the art such as phosphorylation, sulfonation, acylation with, for example, acetyl, pyruvoyl, propionyl, succinyl, lactoyl or 3-hydroxybutyl groups, esterification with, for example, glyceryl, the formation of metallic complexes of the exopolysaccharide and/or chemical sulfation.

The molecular weight of the polysaccharide is comprised between 100,000 and 10 million Da, and more preferably between 100,000 and 5 million Da. In a particular embodiment, a radical depolymerization is carried out wherein the molecular weight is between 100,000 and 1 million Da. Depolymerization methods are known in the prior art, for example and not restricted to those described in *"*Low molecular weight heparins (5 kDa) and oligoheparins (2 kDa) produced by gel permeation enrichment or radical process: Comparison of structures and physicochemical and biological properties.", Volpi et al., Anal. Biochem., (1992), 200, 100-107*].*

The exopolysaccharide produced by the strain of the species *Vibrio sp.* with deposit number CNCM I-4239 is characterized by presenting at least two different neutral monosaccharides and one acid monosaccharide. The neutral monosaccharides are *N*-acetylglucosamine and *N*-acetylgalactosamine. The acid monosaccharide is glucuronic acid. The exopolysaccharide of this invention presents a composition in weight from 30% to 55% of glucuronic acid, from 30 to 60% of *N*-acetylglucosamine, and from 5% to 20% of *N*-acetylgalactosamine with the condition that the sum of the percentages does not exceed 100%.

Preferably, the exopolysaccharide presents a composition in weight from 35% to 50% of glucuronic acid, from 40% to 55% of *N*-acetylglucosamine, and from 5% to 15% of *N-*acetylgalactosamine.

Other aspect of this invention relates to a cosmetic or dermopharmaceutical composition characterized in that it comprises a cosmetically or dermopharmaceutically effective quantity of the exopolysaccharide of this invention and at least one cosmetically and/or dermopharmaceutically acceptable excipient, adjuvant and/or ingredient. Said compositions can be prepared by the conventional methods known by the persons skilled in the art *["*Harry's Cosmeticology", Seventh edition, (1982), Wilkinson J.B., Moore R.J., ed. Longman House, Essex, GB*].*

The cosmetically or dermopharmaceutically effective quantity of the exopolysaccharide in the invention to be administered, as well as its dosage, will depend on numerous factors, including age, condition of the patient, the nature or severity of the condition, disorder or disease to be treated and/or cared for, the route and frequency of administration and the nature, in particular, of the exopolysaccharides to be used.

"Cosmetically or dermopharmaceutically effective" is understood to be a non-toxic but sufficient quantity of the exopolysaccharide to provide the desired effect. The exopolysaccharide of the invention is preferably used, with regard to the total weight of the composition, between 0.00000001% (in weight) and 20% (in weight); preferably between 0.000001% (in weight) and 15% (in weight), more preferably between 0.0001% (in weight) and 10% (in weight) and even more preferably between 0.0001% (in weight) and 5% (in weight).

In a particular embodiment, the exopolysaccharide of the invention can also be incorporated into cosmetic and/or dermopharmaceutical delivery systems and/or sustained release systems.

The term "delivery systems" relates to a cosmetically and/or dermopharmaceutically acceptable carrier such as a diluent, adjuvant, excipient, vehicle or additives with which the exopolysaccharide of the invention is administered. These cosmetic and/or dermopharmaceutical vehicles can be liquids, such as water, oils or surfactants, including those of petroleum, animal, plant or synthetic origin, such as and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin and similar. A person skilled in the art knows the diluents, adjuvants, excipients or additives which can be used in the different delivery systems in which the exopolysaccharide is administered.

The term "sustained release" is used in a conventional sense relating to a delivery system of a compound which provides the gradual release of this compound during a period of time and preferably, although not necessarily, with relatively constant compound release levels over a period of time.

Examples of delivery or sustained release systems are, without limiting sense, liposomes, mixed liposomes, oleosomes, niosomes, ethosomes, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules and nanocapsules, as well as microemulsions and nanoemulsions, which can be added to achieve a greater penetration of the active ingredient. Preferred delivery or sustained release systems are liposomes, surfactant-phospholipid mixed micelles and microemulsions, more preferably water-in-oil microemulsions with an internal reverse micelle structure and nanocapsules containing microemulsions.

The sustained release systems can be prepared by methods known in the prior art, and the compositions which contain them can be administered, for example, by topical or transdermal administration, including adhesive patches, non-adhesive patches, occlusive patches and microelectric patches, and preferably should release a relatively constant quantity of the exopolysaccharide of the invention. The amount of exopolysaccharide contained in the sustained release system will depend, for example, on where the composition is to be administered, the kinetics and duration of the release of the exopolysaccharide of the invention, as well as the nature of the condition, disorder and/or disease to be treated and/or cared for.

The composition containing the exopolysaccharide of this invention can also be adsorbed on solid organic polymers or solid mineral supports, such as and not restricted to, talc, bentonite, silica, starch or maltodextrin among others.

The compositions containing the exopolysaccharide of the invention can also be incorporated into fabrics, non-woven fabrics or medical devices which are in direct contact with the skin, thus releasing the exopolysaccharide of the invention whether by biodegradation of the binding system to the fabric, non-woven fabric or medical device, or due to the friction between them and the body, due to body moisture, the skin's pH or body temperature. Furthermore, the exopolysaccharide of the invention can be incorporated into the fabrics and non-woven fabrics used in the manufacture of garments that are in direct contact with the body. In a preferred embodiment, the fabrics, non-woven fabrics or medical devices which contain the exopolysaccharide are used for the treatment of those conditions, disorders and/or diseases which improve or are prevented by inhibition of neuronal exocytosis, by stimulation of fibroblast proliferation or by healing and/or re-epithelialization of the skin and/or mucous membranes.

Examples of fabrics, non-woven fabrics, garments, medical devices and means for immobilizing the compounds to them, among which are the delivery systems and/or the sustained release systems described above, can be found in literature and are known in the prior art *[*Schaab C.K. 1986 "Impregnating Fabrics With Microcapsules", HAPPI May 1986*;* Nelson G Int. J. Pharm. 2002, 242:55-62*;* Hipler U. C. y Elsner P. 2006, "Biofunctional Textiles and the Skin", Curr. Probl. Dermatol. v.33,., eds. S. Karger AG, Basel, Switzerl*and;* Malcom R.K. et al. J. Cont. Release, 2004, 97:313-320*].* The preferred fabrics, non-woven fabrics, garments and medical devices are bandages, gauzes, t-shirts, socks, tights, underwear, girdles, gloves, diapers, sanitary napkins, dressings, bedspreads, flannels, adhesive patches, non-adhesive patches, occlusive patches, microelectric patches and/or face masks.

The cosmetic or dermopharmaceutical compositions containing the exopolysaccharide of this invention can be used in different types of compositions of topical or transdermal application, optionally including cosmetically and/or dermopharmaceutically acceptable excipients necessary for formulating the desired administration form.

Compositions of topical or transdermal application can be produced in any solid, liquid or semisolid formulation, such as for example and not restricted to, multiple emulsions, such as and not restricted to, creams, multiple emulsions such as for example and not restricted to, oil and/or silicone in water emulsions, water-in-oil and/or silicone emulsions, water/oil/water or water/silicone/water type emulsions, and oil/water/oil or silicone/water/silicone type emulsions, liquid crystals, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These formulations are topically or transdermally applied and can be incorporated using techniques known by the person skilled in the art into different types of solid accessories, such as and not restricted to, bandages, gauzes, t-shirts, socks, tights, underwear, girdles, gloves, diapers, sanitary napkins, dressings, bedspreads, towelettes, adhesive patches, non-adhesive patches, occlusive patches, microelectric patches and/or face masks, or they can be incorporated into different make-up products such as make-up foundation, such as fluid foundations and compact foundations, lotions make-up removal lotions, make-up removal milks, under-eye concealers, eye shadows, lipsticks, lip protectors, lip gloss and powders among others.

The cosmetic or dermopharmaceutical compositions of the invention may include agents which increase the percutaneous absorption of the compounds of this invention, for example and not restricted to, dimethyl sulfoxide, dimethylacetamide, dimethylformamide, surfactants, azone (1-dodecylazacycloheptane-2-one), alcohol, urea, ethoxydiglycol, acetone, propylene glycol or polyethylene glycol, among others. Furthermore, the cosmetic or dermopharmaceutical compositions of this invention can be applied to local areas to be treated by means of iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof, to achieve a greater penetration of the compound of the invention. The application area will be determined by the nature of the condition, disorder and/or disease to be treated and/or cared for.

Among the cosmetically or dermopharmaceutically acceptable excipients, adjuvants and/or ingredients contained in the cosmetic or dermopharmaceutical compositions described in this invention are additional ingredients commonly used in cosmetic or dermopharmaceutical compositions such as and not restricted to, other inhibitors of neuronal exocytosis, other anticholinergic agents, other muscle contraction inhibiting agents, other antiaging agents, other anti-wrinkle agents, other antiperspirant agents, other anti-inflammatory agents and/or analgesics, other anti-itching agents, calming agents, anesthetic agents, agents inhibiting acetylcholine receptor clustering, agents that inhibit acetylcholinesterase, skin relaxant agents, melanin synthesis stimulating or inhibiting agents, whitening or depigmenting agents, propigmenting agents, self-tanning agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin conditioners, humectants, substances that retain moisture, alpha hydroxyacids, beta hydroxyacids, moisturizers, epidermal hydrolytic enzymes, vitamins, amino acids, proteins, pigments or colorants, dyes, biopolymers, gelling polymers, thickeners, surfactants, softening agents, emulsifiers, binding agents, preservatives, agents able to reduce or treat bags under the eyes, exfoliating agents, desquamating agents, keratolytic agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, collagen synthesis-stimulating agents, elastin synthesis-stimulating agents, decorin synthesis-stimulating agents, laminin synthesis-stimulating agents, defensin synthesis-stimulating agents, chaperone synthesis-stimulating agents, cAMP synthesis-stimulating agents, agents that modulate AQP-3, agents that modulate aquaporin synthesis, proteins from the aquaporin family, hyaluronic acid synthesis-stimulating agents, gycosaminoglycan synthesis-stimulating agents, fibronectin synthesis-stimulating agents, sirtuin synthesis-stimulating agents, sirtuin activating agents, heat shock proteins, heat shock protein synthesis-stimulating agents, agents stimulating the synthesis of lipids and components of the stratum corneum, ceramides, fatty acids, agents that inhibit collagen degradation, agents that inhibit matrix metalloproteinases, agents that inhibit elastin degradation, agents that inhibit serine proteases such as kallikreins, leukocyte elastase, cathepsin G, agents stimulating fibroblast proliferation, agents stimulating keratinocyte proliferation, agents stimulating adipocyte proliferation, agents stimulating melanocyte proliferation, agents stimulating keratinocyte differentiation, agents that accelerate or delay adipocyte differentiation, antihyperkeratosis agents, comedolytic agents, anti-psoriasis agents, DNA repairing agents, DNA protecting agents, stem cell protecting agents, stabilizers, agents for the treatment and/or care of sensitive skin, firming agents, anti-stretch mark agents, binding agents, agents regulating sebum production, lipolytic agents or agents stimulating lipolysis, adipogenic agents, agents that modulate PGC-1α expression, agents that modulate PPARγ, agents that increase or reduce the triglyceride content of adipocytes, anti-cellulite agents, PAR-2 activity inhibiting agents, agents stimulating healing, coadjuvant healing agents, agents stimulating reepithelialization, coadjuvant reepithelialization agents, cytokines, growth factors, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, hair loss delaying agents, perfumes, cosmetic deodorant agents and/or body odor absorbent agent and/or body odor masking agent, chelating agents, plant extracts, essential oils, marine extracts, agents obtained from a biotechnological process, mineral salts, cell extracts, sunscreens and organic or mineral photoprotective agents active against ultraviolet A and/or B rays and/or infrared A rays or mixtures thereof, among others, provided that they are physical and chemically compatible with the rest of components of the composition and in particular with the exopolysaccharide of this invention. Furthermore, the nature of these additional ingredients should not unacceptably alter the benefits of the exopolysaccharide of this invention. The nature of said additional ingredients can be synthetic or natural in origin, such as plant extracts, or come from a biotechnological process or a combination of a synthetic process and a biotechnological process. Additional examples can be found described in CTFA International Cosmetic Ingredient Dictionary & Handbook, 12th Edition (2008*).* In the context of this invention, biotechnological process is understood to be any process that produces the active ingredient, or part thereof, in an organism, or in a part thereof.

In a particular embodiment, the anti-wrinkle and/or antiaging agent is selected, for example and not restricted to, from the group formed by extracts or hydrolyzed extracts of *Vitis vinifera, Rosa canina, Curcuma longa, Theobroma cacao, Ginkgo biloba, Leontopodium alpinum* or *Dunaliella salina* among others; Matrixyl^{®} [INCI: Palmitoyl Pentapeptide-4], Matrixyl 3000^{®} [INCI: Palmitoyl Tetrapeptide-7, Palmitoyl Oligopeptide], Matrixyl^{®} Synthe'6 [INCI: Glycerin, Water, Hydroxypropyl Cyclodextrin, Palmitoyl Tripeptide-38], Essenskin™ [INCI: Calcium Hydroxymethionine], Renovage [INCI: Teprenone] or Dermaxyl^{®} [INCI: Palmitoyl Oligopeptide] marketed by Sederma/Croda, Vialox^{®} [INCI: Pentapeptide-3], Syn^{®}-Ake^{®} [INCI: Dipeptide Diaminobutyroyl Benzylamide Diacetate], Syn^{®}-Coll [INCI: Palmitoyl Tripeptide-5], Phytaluronate [INCI: Locust Bean (Ceratonia Siliqua) Gum] or Preregen^{®} [INCI: Glycine Soja (Soybean) Protein, Oxido Reductases] marketed by Pentapharm/DSM, Myoxinol™ [INCI: Hydrolyzed Hibiscus Esculentus Extract], Syniorage™ [INCI: Acetyl Tetrapeptide-11], Dermican™ [INCI: Acetyl Tetrapeptide-9] or DN AGE™ LS [INCI: Cassia Alata leaf Extract] marketed by Laboratoires Sérobiologiques/Cognis/BASF, Algisum C^{®} [INCI: Methylsilanol Mannuronate] or Hydroxyprolisilane CN^{®} [INCI: Methylsilanol Hydroxyproline Aspartate] marketed by Exsymol, Argireline^{®} [INCI: Acetyl Hexapeptide-8], SNAP-7 [INCI: Acetyl Heptapeptide-4], SNAP-8 [INCI: Acetyl Octapeptide-3], Leuphasyl^{®} [INCI: Pentapeptide-18], Inyline™ [INCI: Acetyl Hexapeptide-30], Aldenine^{®} [INCI: Hydrolized Wheat Protein, Hydrolized Soy Protein, Tripeptide-1], Preventhelia™ [INCI: Diaminopropionoyl Tripeptide-33], Decorinyl^{®} [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Eyeseryl^{®} [INCI: Acetyl Tetrapeptide-5], Peptide AC29 [INCI: Acetyl Tripeptide-30 Citrulline], Relistase™ [INCI: Acetylarginyltriptophyl Diphenylglycine], Thermostressine^{®} [INCI: Acetyl Tetrapeptide-22], Lipochroman™ 6 [INCI: Dimethylmethoxy Chromanol], Chromabright™ [INCI: Dimethylmethoxy Chromanyl Palmitate], Antarcticine^{®} [INCI: Pseudoalteromonas Ferment Extract], dGlyage™ [INCI: Lysine HCl, Lecithin, Tripeptide-9 Citrulline], Vilastene™ [INCI: Lysine HCl, Lecithin, Tripeptide 10 Citrulline], Hyadisine^{®} [INCI: Pseudoalteromonas Ferment Extract], Hyanify™ [INCI: Saccharide Isomerate], Diffuporine™ [INCI: Acetyl Hexapeptide-37], Silusyne™ [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39] or Adifyline™[INCI: Acetyl Hexapeptide-38] marketed by Lipotec/Lubrizol, Kollaren^{®} [INCI: Tripeptide 1, Dextran] marketed by Institut Europeen de Biologie Cellulaire, Collaxyl^{®} IS [INCI: Hexapeptide-9], Laminixyl IS™ [INCI: Heptapeptide], Orsirtine™ GL [INCI: Oryza Sativa (Rice) Extract], D'Orientine™ IS [INCI: Phoenix Dactylifera (Date) Seed Extract], Phytoquintescine™ [INCI: Einkorn (Triticum Monococcum) Extract] or Quintescine™ IS [INCI: Dipeptide-4] marketed by Vincience/ISP/Ashland, BONT-L-Peptide [INCI: Palmitoyl Hexapeptide-19] marketed by Infinitec Activos, Deepaline™ PVB [INCI: Palmitoyl hydrolyzed Wheat Protein] or Sepilift^{®} DPHP [INCI: Dipalmitoyl Hydroxyproline] marketed by Seppic, Gatuline^{®} Expression [INCI: Acmella Oleracea Extract], Gatuline^{®} In-Tense [INCI: Spilanthes Acmella Flower Extract] or Gatuline^{®} Age Defense 2 [INCI: Juglans Regia (Walnut) Seed Extract] marketed by Gattefossé, Thalassine™ [INCI: Algae Extract] marketed by Biotechmarine, ChroNOline™ [INCI: Caprooyl Tetrapeptide-3] or Thymulen-4 [INCI: Acetyl Tetrapeptide-2] marketed by Atrium/Unipex Innovations, EquiStat [INCI: Pyrus Malus Fruit Extract, Glycine Soja Seed Extract] or Juvenesce [INCI: Ethoxydiglicol and Caprylic Triglycerid, Retinol, Ursolic Acid, Phytonadione, Ilomastat] marketed by Coletica/Engelhard/BASF, Ameliox [INCI: Carnosine, Tocopherol, Silybum Marianum Fruit Extract] or PhytoCellTec Malus Domestica [INCI: Malus Domestica Fruit Cell Culture] marketed by Mibelle Biochemistry, Bioxilift [INCI: Pimpinella Anisum Extract] or SMS Anti-Wrinkle^{®} [INCI: Annona Squamosa Seed Extract] marketed by Silab, antagonists of the Ca²⁺ channel such as and not restricted to, alverine, manganese or magnesium salts, certain secondary or tertiary amines, retinol and its derivatives, idebenone and its derivatives, Coenzyme Q10 and its derivatives, boswellic acid and its derivatives, GHK and its derivatives and/or salts, carnosine and its derivatives, DNA repairing enzymes such as and not restricted to, photolyase or T4 endonuclease V, or chloride channel agonists, among others.

In a particular embodiment, the anti-itching agent is selected for example and not restricted to, extracts of *Abelmoschus esculentus, Actaea alba, Aglaia odorata, Alkanna tinctoria, Althaea officinalis, Altingia excelsa, Andropogon virginicus, Aralia nudicaulis, Aralia racemosa, Argemone mexicana, Barleria prionitis, Camelia sinensis, Caesalpinia digyna, Campsis grandiflora, Carissa congesta, Carthamus oxyacantha, Cassia tora, Chrysanthemum indicum, Cimicifuga racemosa, Cinnamomum camphora, Clematis vitalba, Cuscuta reflexa, Diospyros peregrina, Enicostema axillare, Hammamelis virginiana, Jatropha multifida, Lavandula officinalis, Lavandula latifolia, Liquidambar orientalis, Lithospermum officinale, Madhuca longifolia, Martynia annua, Medicago sativa, Michelia champaca, Mikania glomerata, Mimosa pudica, Oryza sativa, Phaseolus vulgaris, Phyllanthus urinaria, Phyllanthus virgatus, Pistacia vera, Polygonum hydropiper, Quercus ilex, Rauvolfia caffra, Ricinus communis, Rubus idaeus, Sagittaria sagittifolia, Sandoricum koetjape, Sapindus mukorossi, Schleichera oleosa, Sesbania grandiflora, Spondias dulcis, Tilia sp., Toona ciliata, Tragia involucrata, Trichosanthes quinquangulata, Vaccaria pyramidata, Ventilago madraspatana, Veratrum album* or *Xanthium strumarium* among others or as well as one synthetic compound or product of biotechnological origin which is an anti-itching agent, for example and not restricted to, Neutrazen™ [INCI: Water, Butylene Glycol, Dextran, Palmitoyl Tripeptide-8] marketed by Atrium /Unipex Innovations, Meliprene^{®} [INCI: Dextran, Acetil Heptapeptide-1] marketed by Institut Européen de Biologie Cellulaire/Unipex Innovations, Delisens™ [proposed INCI: Acetyl Hexapeptide-46] marketed by Lipotec/Lubrizol, Skinasensyl™ [INCI: Acetyl Tetrapeptide-15] marketed by Laboratoires Sérobiologiques/Cognis/BASF, SymSitive^{®} 1609 [INCI: 4-t-Butylcyclohexanol] marketed by Symrise, Symbiocell™ [INCI: Extract from Cestrum Latifolium] marketed by BASF, Gatuline^{®} Derma-Sensitive [INCI: Octyldodecyl Myristate, Capparis Spinosa Fruit Extract] marketed by Gattefossé or MAXnolia [INCI: Magnolia Officinalis Bark Extract, Vitis Vinifera/Vitis Vinifera (Grape) Seed Extract, Tocopherol] marketed by Mibelle Biochemistry among others, or mixtures thereof.

In another particular embodiment, then anti-inflammatory agent and/or analgesic agent is selected, for example and not restricted to, from the group formed by extract of madecassoside, extract of echinacea, amaranth seed oil, sandal wood oil, extract of peach tree leaf, extract of *Aloe vera, Arnica montana, Artemisia vulgaris, Asarum maximum, Calendula officinalis, Capsicum, Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Origanum vulgare, Prunus laurocerasus, Rosmarinus officialis, Salix alba, Silybum marianum, Tanacetum parthenium, Thymus vulgaris, Uncaria guianensis* or *Vaccinum myrtillus,* omega-3 and omega-6 fatty acids, Neutrazen™ [INCI: Water, Butylene Glycol, Dextran, Palmitoyl Tripeptide-8] marketed by Atrium/Unipex Group, Delisens™ [proposed INCI: Acetyl Hexapeptide-46] marketed by Lipotec/Lubrizol, Meliprene^{®} [INCI: Dextran, Acetyl Heptapeptide-1] marketed by Institut Européen de Biologie Cellulaire/Unipex Innovations, Skinasensyl™ [INCI: Acetyl Tetrapeptide-15] or Anasensyl™ [INCI: Mannitol, Ammonium Glycyrrhizate, Caffeine, Hippocastanum (Horse Chestnut) Extract] marketed by Laboratoires Serobiologiques/Cognis/BASF, Calmosensine™ [INCI: Acetyl Dipeptide-1] marketed by Sederma/Croda, coenzyme Q10 or alkyl glyceryl ethers, among others, or mixtures thereof.

In another particular embodiment, the agent which inhibits neuronal exocytosis, anticholinergic agent, inhibitor of acetylcholine-receptor clustering and/or a muscle contraction inhibitor is selected, for example and not restricted to, from the group formed by extracts of *Atropa belladonna, Hyoscyamus niger, Mandragora officinarum, Chondodendron tomentosum,* plants from the *Brugmansias* genus, or from the *Daturas* genus, *Clostridium botulinum* toxin, peptides derived from the protein SNAP-25, peptides derived from the protein synaptotagmin, peptides derived from the protein syntaxin, peptides derived from the protein synaptobrevin, peptides derived from the protein snapin, Argireline^{®} [INCI: Acetyl Hexapeptide-8], SNAP-7 [INCI: Acetyl Heptapeptide-4], SNAP-8 [INCI: Acetyl Octapeptide-3], Leuphasyl^{®} [INCI: Pentapeptide-18] or Inyline™ [INCI: Acetyl Hexapeptide-30] marketed by Lipotec/Lubrizol, BONT-L-Peptide [INCI: Palmitoyl Hexapeptide-19] marketed by Infinitec Activos, and Vialox^{®} [INCI: Pentapeptide-3] or Syn^{®} Ake^{®} [INCI: Dipeptide Diaminobutyroyl Benzylamide Diacetate] marketed by Pentapharm/DSM among others, or mixtures thereof.

In a particular embodiment, the firming and/or redensifying and/or restructuring agent is selected, for example and not restricted to, from the group formed by extracts of *Malpighia punicitolia, Cynara scolymus, Gossypium herbaceum, Aloe Barbadensis, Panicum miliaceum, Morus nigra, Sesamum indicum, Glycine soja, Triticum vulgare,* Pronalen^{®} Refirming HSC [INCI: Triticum Vulgare, Silybum Marianum, Glycine Soy, Equisetum Arvense, Alchemilla Vulgaris, Medicago Sativa, Raphanus Sativus] or Polyplant^{®} Refirming [INCI: Coneflower, Asiatic Centella, Fucus, Fenugreek] marketed by Provital, Lanablue^{®} [INCI: Sorbitol, Algae Extract] marketed by Atrium Biotechnologies/Unipex Innovations, Pepha^{®}-Nutrix [INCI: Natural Nutrition Factor] marketed by Pentapharm/DSM, plant extracts containing isoflavones, Biopeptide EL™ [INCI: Palmitoyl Oligopeptide], Biopeptide CL™ [INCI: Palmitoyl Oligopeptide], Vexel^{®} [INCI: Water (Aqua), Propylene Glycol, Lecithin, Caffeine, Palmitoyl Carnitine], Matrixyl^{®} [INCI: Palmitoyl Pentapeptide-3], Matrixyl^{®} 3000 [INCI: Palmitoyl Tetrapeptide-3, Palmitoyl Oligopeptide] or Bio-Bustyl™ [INCI: Glyceryl Polymethacrylate, Rahnella Soy Protein Ferment, Water (Aqua), Propylene Glycol, Glycerin, PEG-8, Palmitoyl Oligopeptide] marketed by Sederma/Croda, Dermosaccharides^{®} HC [INCI: Glycerin, Water (Aqua), Glycosaminoglycans, Glycogen], Aglycal^{®} [INCI: Mannitol, Cyclodextrin, Glycogen, Aratostaphylos Uva Ursi Leaf Extract], Cytokinol^{®} LS [INCI: Hydrolyzed Casein, Hydrolyzed Yeast Protein, Lysine HCl] or Firmiderm^{®} LS9120 [INCI: Terminalia Catappa Leaf Extract, Sambucus Negra Flower Extract, PVP, Tannic Acid] marketed by Laboratoires Serobiologiques/Cognis/BASF, Liftline^{®} [INCI: Hydrolyzed Wheat Protein], Raffermine^{®} [INCI: Hydrolyzed Soy Flour] or Ridulisse C^{®} [Hydrolyzed Soy Protein] marketed by Silab, Serilesine^{®} [INCI: Hexapeptide-10], Decorinyl™ [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Silusyne™ [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39] or Adifyline™[INCI: Acetyl Hexapeptide-38] marketed by Lipotec/Lubrizol, Ursolisome^{®} [INCI: Lecithin, Ursolic Acid, Atelocollagen, Xanthan Gum, Sodium Chondroitin Sulfate] or Collalift^{®} [INCI: Hydrolyzed Malt Extract] marketed by Coletica/Engelhard/BASF, Syn^{®}-Coll [INCI: Palmitoyl Tripeptide-5] marketed by Pentapharm/DSM, Hydriame^{®} [INCI: Water (Aqua), Glycosaminoglycans, Sclerotium Gum] marketed by Atrium Biotechnologies/Unipex Innovations or IP2000 [INCI: Dextran, Trifluoroacetyl Tripeptide-2] marketed by Institut Europeen de Biologie Cellulaire/Unipex Innovations, among others.

In a particular embodiment, the agent stimulating the synthesis of dermal or epidermal macromolecules is selected, for example and not restricted to, from the group formed by collagen synthesis-stimulating agents, elastin synthesis-stimulating agents, decorin synthesis-stimulating agents, laminin synthesis-stimulating agents, chaperone synthesis-stimulating agents, sirtuin synthesis-stimulating agents, sirtuin activating agents, aquaporin synthesis-modulating agents, fibronectin synthesis-stimulating agent, agents that inhibit collagen degradation, agents that inhibit elastin degradation, agents that inhibit serine proteases such as kallikreins, leukocyte elastase or cathepsin G, agents stimulating fibroblast proliferation, agents stimulating adipocyte proliferation, agents that accelerate or delay adipocyte differentiation, and DNA repairing agents and/or DNA protecting agents, such as and not restricted to extracts of *Centella asiatica, Saccharomyces cerivisiae, Solanum tuberosum, Rosmarinus officinalis, Vaccinium angustifolium,* extract of the algae *Macrocystis pyrifera, Padina pavonica,* extract of soy, malt, flax, sage, red clover, kakkon, white lupin plants, hazelnut extract, maize extract, yeast extract, beech shoot extracts, leguminous seed extract, plant hormone extract such as gibberellins, auxins or cytokinins, among others, or extract of zooplankton Salina, fermentation product of milk with *Lactobacillus Bulgaricus,* asiaticosides and their derivatives, vitamin C and its derivatives, cinnamic acid and its derivatives, Matrixyl^{®} [INCI: Palmitoyl Pentapeptide-3], Matrixyl^{®} 3000 [INCI: Palmitoyl Tetrapeptide-3, Palmitoyl Oligopeptide] or Biopeptide CL™ [INCI: Glyceryl Polymethacrylate, Propylene Glycol, Palmitoyl Oligopeptide] marketed by Sederma/Croda, Antarcticine^{®} [INCI: Pseudoalteromonas Ferment Extract], Decorinyl^{®} [INCI: Tripeptide-10 Citrulline], Serilesine^{®} [INCI: Hexapeptide-10], Lipeptide [INCI: Hydrolized Vegetable Protein], Aldenine^{®} [INCI: Hydrolized Wheat Protein, Hydrolized Soy Protein, Tripeptide-1], Relistase™ [INCI: Acetylarginyltriptophyl Diphenylglycine], Thermostressine™ [INCI: Acetyl Tetrapeptide-22], Peptide AC29 [INCI: Acetyl Tripeptide-30 Citrulline], Diffuporine™ [INCI: Acetyl Hexapeptide-37], Silusyne™ [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39] or Adifyline™[INCI: Acetyl Hexapeptide-38] marketed by Lipotec/Lubrizol, Drieline^{®} PF [INCI:Yeast Betaglucan] marketed by Alban Muller, Phytovityl C^{®} [INCI: Aqua, Zea Mays Extract] comercializado por Solabia, Collalift^{®} [INCI: Hydrolyzed Malt Extract] marketed by Coletica/Engelhard/BASF, Phytocohesine PSP™ [INCI: Sodium Beta-Sitosterol Sulfate] marketed by Vincience/ISP/Ashland, minerals such as calcium, among others, isoflavonoids, carotenoids, in particular lycopene, pseudodipeptides, retinoids and their derivatives such as retinol or retinyl palmitate, among others, or heparinoids, among others.

In another particular embodiment, the agent stimulating healing, coadjuvant healing agent, agent stimulating reepithelialization and/or coadjuvant reepithelialization agent is selected, for example and not restricted to, from the group formed by extracts of *Aristoloquia clematis, Centella asiatica, Rosa moschata, Echinacea angustifolia, Symphytum officinal, Equisetum arvense, Hypericum perforatum, Mimosa tenuiflora, Persea gratísima, Prunus africanum, Tormentilla erecta, Aloe vera,* Polyplant^{®} Epithelizing [INCI: Calendula Officinalis, Hypericum Perforatum, Chamomilla Recutita, Rosmarinus Officinalis] marketed by Provital, Cytokinol^{®} LS 9028 [INCI: Hydrolyzed Casein, Hydrolyzed Yeast Protein, Lysine HCl] marketed by Laboratories Serobiologiques/Cognis/BASF or Deliner^{®} [INCI: Zea Mays (Corn) Kernel Extract] marketed by Coletica/Engelhard, allantoin, cadherins, integrins, selectins, hyaluronic acid receptors, immunoglobulins, fibroblast growth factors, connective tissue growth factors, platelet-derived growth factors, vascular endothelial growth factors, epidermal growth factors, insulin-like growth factor, keratinocyte growth factors, colony-stimulating factors, transforming growth factor-beta, tumor necrosis factor-alpha, interferons, interleukins, matrix metalloproteases, receptors of protein tyrosine phosphatase, Antarcticine^{®} [INCI: Pseudoalteromonas Ferment extract], Bodyfensine^{®} [INCI: Acetyl Dipeptide-3 Aminohexanoate] or Decorinyl™ [INCI: Tripeptide 10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Xpertmoist™ [INCI: Glycerin, Pseudoalteromonas Ferment Extract, Xanthan Gum, Proline, Alanine, Serine, Ethylhexylglycerin, Caprylyl Glycol], Serilesine^{®} [INCI: Hexapeptide-10] or Thermostressine™ [INCI: Acetyl Tetrapeptide-22], marketed by Lipotec/Lubrizol, among others, and/or mixtures thereof.

In another particular embodiment, the cosmetic deodorant agent and/or body odor absorbent agent and/or body odor masking agent and/or antiperspirant agent, perfume and/or perfumed oil is selected, for example and not restricted to, from the group formed by the complex zinc salt of ricinoleic acid, derivatives of abiotic acid, salvia essence, chamomile essence, carnation essence, lemon balm essence, mint essence, cinnamon leaf essence, lime blossom essence, juniper berry essence, vetiver essence, frankincense essence, galbanum essence, labdanum essence, lavender essence, peppermint essence, benzoin, bergamot, dihydromyrcenol, lilial, lyral, citronellol, lemon essence, mandarin essence, orange essence, muscatel, geranium bourbon essence, aniseed, cilantro, cumin, juniper, extracts of fleur-delis, lily, roses, jasmine, neroli; benzyl acetate, *p-tert*-butylcyclohexyl acetate, linalyl acetate, phenylethyl acetate, ethylmethylphenyl glycinate, linalyl benzoate, benzyl formiate, alylcyclohexyl propionate, stiralyl propionate, benzyl salicylate, benzylethylether, linear alkanals with from 8 to 18 carbon atoms, citral, ricinoleic acid, citronellal, citronellyloxyacetaldehyde, cyclamen aldehyde, hydroxycitronellal, bourgeonal, ionones, methyl cedryl ketone, anethole, eugenol, isoeugenol, geraniol, linalool, terpineol, phenylethylalcohol, α-hexylcinnamaldehyde, geraniol, benzylacetone, cyclamenaldehyde, ambroxan, indole, hedione, sandelice, cyclovertal, β-damascone, allylamyl glycolate, dihydromyrcenol, phenoxyethylene isobutyrate, cyclohexyl salicylate, phenylacetic acid, geranyl acetate, romilate, irotyl, floramate, aluminum salts such as alum, aluminum chloride, aluminum chlorohydrate, aluminum dichlorohydrate, aluminum sesquichlorohydrate, aluminum hydroxy allantoinate, aluminum chlorotartrate, aluminum and zirconium trichlorohydrate, aluminum and zirconium tetrachlorohydrate, aluminum and zirconium pentachlorohydrate and/or mixtures thereof, Leuphasyl^{®} [INCI: Pentapeptide-18], SNAP-7 [INCI: Acetyl Heptapeptide-4], SNAP-8 [INCI: Acetyl Octapeptide-3], Argireline^{®} [INCI: Acetyl Hexapeptide-8] or Inyline™ [INCI: Acetyl Hexapeptide-30] marketed by Lipotec/Lubrizol, Vialox^{®} [INCI: Pentapeptide 3] or Syn^{®} Ake^{®} [INCI: Dipeptide Diaminobutyroyl Benzylamide Diacetate] marketed by Pentapharm/DSM and BONT-L-Peptide [INCI: Palmitoyl Hexapeptide-19] marketed by Infinitec Activos among others, or mixtures thereof.

### Applications

In another aspect, this invention refers to the use of the exopolysaccharide of the strain of the species *Vibrio sp.* with deposit number CNCM I-4239, in the preparation of a cosmetic or dermopharmaceutical composition for the treatment and/or care of the skin, mucous membranes, hair and/or nails. In particular, the treatment relates to the treatment and/or prevention of the pain, inflammation, itching or hyperhidrosis, re-epithelizing and/or healing treatment of the skin and/or mucous membranes, treatment and/or prevention of skin aging, treatment and/or prevention of skin wrinkles, preferably expression wrinkles, for the treatment and/or prevention of loss of skin firmness, for the treatment and/or prevention of hyperhidrosis or perspiration, treatment and/or care of skin disorders selected from the group formed by warts, calluses, treatment stimulating hair growth and/or prevention of hair loss.

In another particular embodiment, this invention refers to the use of the exopolysaccharide of the strain of the species *Vibrio sp.* with deposit number CNCM I-4239 in the preparation of a cosmetic or dermopharmaceutical composition for the inhibition of neuronal exocytosis.

In another particular embodiment, this invention refers to the use of the exopolysaccharide of the strain of the species *Vibrio sp.* with deposit number CNCM I-4239 in the preparation of a cosmetic or dermopharmaceutical composition for the stimulation of fibroblast proliferation.

An additional aspect of this invention refers to a method of treatment and/or care of the skin, mucous membranes, hair and/or nails which comprises the administration of a cosmetically and/or dermopharmaceutically effective quantity of the exopolysaccharide of the strain of the species *Vibrio sp.* with deposit number CNCM I-4239. In particular, the treatment relates to the treatment and/or prevention of the pain, inflammation, itching or hyperhidrosis, re-epithelizing and/or healing treatment of the skin and/or mucous membranes, treatment and/or prevention of skin aging, treatment and/or prevention of skin wrinkles, preferably expression wrinkles, for the treatment and/or prevention of loss of skin firmness, for the treatment and/or prevention of hyperhidrosis or perspiration, treatment and/or care of skin disorders selected from the group formed by warts, calluses, treatment stimulating hair growth and/or prevention of hair loss.

In a preferred embodiment, the pain is selected from pain associated with conditions, diseases and/or disorders, for example and not restricted to, touch sensitivity, sensitivity to cold, sensitivity to heat, cutaneous irritation, post-hair removal cutaneous irritation, post-shaving cutaneous irritation, psoriasis, sensitive skin, dermatitis, atopic dermatitis, contact dermatitis, diaper dermatitis, seborrheic dermatitis, eczema, lichen planus, burns, sunburn, arthritis, rheumatoid arthritis, osteoarthritis, psoriatic arthritis, hypersensitivity, cutaneous pain or irritation after surgery, after intense pulsed light treatment (IPL), after treatment with monochromatic pulsed light (laser), after a treatment with chemical desquamating agents or after overexposure to external aggressive agents, among others.

In another preferred embodiment, the inflammation is selected from, for example and not restricted to, psoriasis, sensitive skin, dermatitis, atopic dermatitis, contact dermatitis, diaper dermatitis, seborrheic dermatitis, eczema, rosacea, acne, hyperproliferative skin disease, burns, sunburn, paronychia, cutaneous inflammation after surgery, after intense pulsed light treatment (IPL), after treatment with monochromatic pulsed light (laser), after a treatment with chemical desquamating agents or after overexposure to external aggressive agents, inflammation of the mucous membrane of the vagina, inflammation of the oral mucous membranes, gingivitis, periodontitis, rhinitis, allergic rhinitis, among others.

In another preferred embodiment, the itching is selected from itching associated with conditions, diseases and/or disorders, for example and not restricted to, dermatitis, atopic dermatitis, contact dermatitis, diaper dermatitis, dermatitis herpetiformis, photodermatosis, photosensitivity, pregnancy related dermatitis, menopause related dermatitis, eczema, sensitive skin, psoriasis, chickenpox, herpes, herpes zoster, Netherton's syndrome, peeling skin syndrome, lichen planus, acne, dandruff, seborrhea, seborrheic dermatitis, alopecia, athlete's foot, candidiasis, hemorrhoids, vaginal itching, perianal itching, anogenital itching, sunburn, hives, pruritic otitis, itchy eyes, senile pruritus, aquagenic pruritus, prurigo nodularis, prurigo planus, pityriasis rosea, xerosis and dry skin, allergic reactions, allergies to medicines, food allergies, allergies to chemical products, exposure to poisonous plants and exposure to insect bites, among others.

Preferably, the treatment and/or prevention of the hyperhidrosis or perspiration, is a treatment and/or prevention of the axillary, facial, genital, palmar or plantar hyperhidrosis or perspiration In another aspect, this invention refers to a method of inhibition of neuronal exocytosis which comprises the administration of a cosmetically and/or dermopharmaceutically effective quantity of the exopolysaccharide of the strain of the species *Vibrio sp.* with deposit number CNCM I-4239.

In another aspect, this invention refers to a method of stimulation of fibroblast proliferation which comprises the administration of a cosmetically and/or dermopharmaceutically effective quantity of the exopolysaccharide of the strain of the species *Vibrio sp.* with deposit number CNCM I-4239.

The administration of the exopolysaccharide of the strain of the species *Vibrio sp.* with deposit number CNCM I-4239 is carried out topically or transdermally. In a more particular aspect topical or transdermal application is carried out by iontophoresis, sonophoresis, electroporation, mechanical pressure, osmotic pressure gradient, occlusive cure, microinjections, by needle-free injections by means of pressure, by microelectric patches, face masks or any combination thereof.

The frequency of the application can vary widely, depending on the needs of each subject, suggesting a range of application from once per month to ten times per day, preferably from once per week to four times per day, more preferably from three times per week to twice per day, even more preferably once per day.

This invention is understood more clearly with the help of the following examples, without limitation and included for illustrative purposes only which describe the preparation and characterization of exopolysaccharide and compositions containing it in accordance with the invention.

### EXAMPLES

### Example 1: Preparation and isolation of the exopolysaccharide excreted by the strain of the species Vibrio sp. with deposit number CNCM I-4239.

### a) Method of culture of strain the species Vibrio sp. with deposit number CNCM 1-4239.

The strain of the species *Vibrio sp.* with deposit number CNCM I-4239 was cultured in a fermenter, at 29°C and at a pH of 7.5, whose broth contained 2216E medium (ZoBell C.E. J. Mar. Res., 1941, 4:42.) enriched with glucose (20 g/l). An inoculum was prepared with 10% (v/v) of a pre-culture and the duration of the fermentation was extended to 72 hours. The speed of aeration and stirring was 2 vvm and 250 rpm, respectively.

### b) Purification of the exopolysaccharide.

The bacteria were separated from the broth by centrifugation at 12,000 g for 45 mins. The polysaccharide was purified with distilled water by ultrafiltration with a polyethersulfone membrane for polysaccharides of over 100 KDa in molecular weight. The average molecular weight of the resulting polysaccharide was 1.8 million Da.

### Example 2: Physical-chemical characterization of the exopolysaccharide produced by the strain of the species Vibrio sp. with deposit number CNCM I-4239.

The content of neutral and acid monosaccharides of the exopolysaccharide obtained according to example 1 was determined by hydrolysis and chromatography of gases according to the method described by Kamerling et al. Biochem. J., 1975 151:491-495*,* and modified by Montreuil et al. in 1986, Glycoproteins. In Carbohydrate analysis: a practical approach. Eds Chaplin et Kennedy, I.R.L Press, Oxford, Washington D.C., pp143-204*.* The percentual relationship of sugars obtained was 47.7% of *N*-acetylglucosamine, 11.4% *N-*acetylgalactosamine and 40.9% of glucuronic acid.

### Example 3: In-vitro healing test with human keratinocytes.

Starting from a culture of human keratinocytes grown to confluence, a treatment with trypsin was carried out and a re-seeding was performed in 48-well plate at 5 x10⁴ cells/well. After 48 hours of incubation at 37°C, 5% CO₂, humidified atmosphere, a cell free area was created by scraping with a pipette tip. Then, the culture medium with the exopolysaccharide produced by the strain of the *Vibrio sp* species with deposit number CNCM I-4239 at a concentration of 0.5 mg/mL was added to the cells. Untreated cells, not treated with any product, were used as negative control, while cells treated with DMEM (Dulbecco's Modified Eagle's Medium) and foetal bovine serum were used as positive controls. At this point the cell-free areas were photographed using a Zeiss Axiovert 40 CFL microscope and AxioCam MRC5 camera. Then, the cultures were incubated (again at 37°C, 5% CO₂, with humidified atmosphere) for 48 hours to allow the cells to migrate to the cell-free area. After this period, new photographs of the cultures were taken, the percentage of healing was calculated compared with time zero, by the increase of area occupied by cells in respect of the initially occupied area.

Table 1 shows the healing increase observed in respect of the negative controls.

**Table 1**

| **Tested products** | **Healing increase in respect to negative control (%)** |
|---|---|
| Negative control | 0% |
| Positive control | 115% |
| Exopolysaccharide excreted by the strain CNCM I-4239 | 92% |

### Example 4: In-vitro proliferation assay on human dermal fibroblasts

Cell proliferation was evaluated by a method of cell viability based on fluorescence measurements. Live cells were distinguished by the presence of intracellular esterase activity, determined by the enzymatic conversion of non-fluorescent compound of calcein-AM which permeated into the cells where it was converted into intensely fluorescent calcein, which was retained inside cells, and which conferred a high green fluorescence intensity.

Human dermal fibroblasts were treated with trypsine and seeded at a density of 5 x 10³ cells/well in 96-well plates. After 24 hours of incubation at 37°C, 5% CO₂, humidified atmosphere, fresh medium was added containing in each well the exopolysaccharide produced by the strain of the *Vibrio sp.* species with deposit number CNCM I-4239 at concentrations of 1 mg/mL, 0.5 mg/mL, and 0.25 mg/mL. Cells not treated with the exopolysaccharide of the invention were used as controls. The cells were incubated for additional 24 hours at 37°C, 5% CO₂, humidified atmosphere. Next, the medium in each well was replaced with 100 µL of calcein-AM (Molecular Probes) in PBS (Phosphate Buffered Saline, Sigma) following the method described by Lynch et al *[*Lynch C.N., Wang Y.C., Lund J.K., Chen Y. - W., Leal J.A., Wiley S.R., "TWEAK induce angiogenesis and proliferation of endothelial cells", J. Cell Biol. 1999; 274(13): 8455-8459*].* The fluorescence was measured at λ_{exc} = 485 nm and λₑₘ = 530 nm in a multiwall plate reader (1420 VICTOR2, EG&G Wallac). Proliferation was calculated as: T/C x 100, where T represents the absorbance of the wells tested and C the absorbance of the control wells.

The results obtained in the assay with calcein-AM are shown in Table 2:

**Table 2**

| **Tested products** | **Growth in respect to the control (%)** |
|---|---|
| Control | 100% |
| Exopolysaccharide excreted by the strain CNCM I-4239 1 mg /mL | 113.6 |
| Exopolysaccharide excreted by the strain CNCM I-4239 0,5 mg/mL | 115.4 |
| Exopolysaccharide excreted by the strain CNCM I-4239 0,25 mg/mL | 118.4 |

### Example 5: Study of the inhibition of the formation of SNARE complex by ELISA as method of detection

With the aim of determining the capacity of inhibition of the formation of the SNARE complex by the exopolysaccharide of the invention, the competitive inhibition by this compound with regards to the formation of this complex was studied compared to SNAP-25. The proportion of SNARE complex formed was determined by the ELISA technique, using one of the proteins from the complex bound to GST.

In a 96-well plate VAMP was immobilized (using a 0.037 µM solution) and subsequently the free spaces were blocked with BSA (Bovine Serum Albumine) (3%). Parallel to this process, SNAP-25 bound to GST (Glutation S-transferase) (0.0185 µM), syntaxin (0.037 µM) and the exopolysaccharide produced by the strain of the species *Vibrio sp.* with deposit number CNCM I-4239 was tested (at 1 mg/mL, 0.5 mg/mL and 0.1 mg/mL) were incubated for 1 hour.

After incubation, the samples were transferred to a plate with immobilized VAMP and were incubated for 1 hour to allow the formation of the SNARE complex. Afterwards, the plate was washed and the complex was detected by a primary antibody anti-GST (Antibody anti-GST epitope TAG, Fisher Cat. no:PA1-982A). The absorbance was readed at a wavelength of 490 nm in a TECAN GENios spectrophotometric reader.

To facilitate the test product completition with one of the proteins forming the SNARE complex (SNAP-25), the proportion of such protein while the rest of proteins are mixed at equimolar concentrations.

Table 3 showns the results of the competitive inhibition for the formation of the SNARE complex by the exopolysaccharide of the invention versus SNAP-25. The percentage of inhibition of the formation of the complex is inversely proportional to the quantity of SNARE complex spectrophotometrically detected.

**Table 3**

| **Tested product** | **% Inhibition formation of SNARE complex** | | |
|---|---|---|---|
| | 1.0 mg/mL | 0.1 mg/mL | 0.5 mg/mL |
| Exopolysaccharide excreted by the strain CNCM I-4239 | 57 | 31 | 53- |

### Example 6: Study of the inhibition of the formation of SNARE complex by electrophoresis as method of detection

VAMP (6 µM), syntaxin (6 µM) and the exopolysaccharide produced by the strain of the species *Vibrio sp.* with deposit number CNCM I-4239 (at 1 mg/mL and 0.1 mg/mL) were incubated for 3 hours. The same dilution generated for the tested exopolysaccharide was generated in the negative control of inhibition of the formation of the complex with ultrapure water (18.2mΩ). Subsequently, SNAP-25 (0.6 µM) was added and the mixture was incubated for additional 15 hours to allow the formation of the SNARE complex. After incubation, the loading buffer (Laemli Simple Buffer) was added and the mixture was analyzed with a gel SDS-PAGE in a gel of 10% acrylamide. The amount of complex was determined by an image acquisition and analysis software.

Table 4 shows the results of the inhibition of the formation of the SNARE complex. The percentage of inhibition of the formation of the complex is inversely proportional to the quantity of SNARE complex detected.

**Table 4**

| **Tested products** | **% inhibition formation SNARE complex** | |
|---|---|---|
| | 1.0 mg/mL | 0.1 mg/mL |
| Exopolysaccharide excreted by the strain CNCM I-4277 | 60 | 6 |

### Ejemplo 7: Quantification of the release of noradrenaline induced by TPA/ionomycin in a neuroblastoma cell line by an ELISA method.

The induction of the release of noradrenaline with TPA (12-O-tetradecanoylphorbol-13-acetate)/Ionomycin enables direct measurement of neuronal exocytosis. For the study of the inhibitory effect of the exopolysaccharide of the invention on the release of noradrenaline, cells of a human neuroblastoma cell line were pre-incubated (1x10⁶ cells/well) for 60 minutes with the exopolysaccharide produced by the strain of the species *Vibrio sp.* with deposit number CNCM I-4239 was tested at concentrations of 2 mg/mL, 1 mg/mL and 0.1 mg/mL. Then, the release of the noradrenaline neurotransmitter was induced by an 8 minute pretreatment with a solution of 12-*O*-tetradecanoylphorbol-13-acetate (TPA) 100 nM, which mobilized the intracellular vesicles which contained the neurotransmitter,followed by a 5 minute incubation with TPA/Ionomycin (100 nM/10 µM), which induced the release of the neurotransmitter contained in these vesicles. The quantity of neurotransmitter released into the culture medium was quantified by ELISA (Noradrenaline ELISA kit, IBL International ref. RE59261), in an assay mediated by specific antibodies against noradrenaline and completed by an enzymatic reaction based on the reaction of alkaline phosphatase, which resulted in a quantifiable color absorbance signal at 405 nm measured in a Thermo Scientific Multiskan Ascent equipment.

The blocking of the SNARE complex by the exopolysaccharide of the invention lead to an inhibition of neuronal exocytosis and therefore, a decrease in the levels of released noradrenaline (Table 5).

**Table 5**

| **Tested products** | | **% RELEASED NORADRENALINE** |
|---|---|---|
| Release positive control (TPA / ION) | | 100 |
| Exopolysaccharide excreted by the strain CNCM I-4239 | 2.0 mg/mL | 65.5 |
| | 1.0 mg/mL | 83.4 |
| | 0.5 mg/mL | 92.1 |

### Example 8: Preparation of a cosmetic composition of the exopolysaccharide excreted by the strain of the species Vibrio sp. with deposit number CNCM I-4239.

A solution at 1% by weight of the exopolysaccharide excreted by the strain of the species *Vibrio sp.* with deposit number CNCM I-4239 was prepared in water [INCI: WATER (AQUA)] with disodium hydrogen phosphate [INCI: DISODIUM PHOSPHATE], sodium phosphate 2-hydrate [INCI: SODIUM PHOSPHATE], Zemea^{®} propanediol [INCI:PROPANEDIOL], Dermosoft^{®} GMCY [INCI: GLYCERYL CAPRYLATE] and xanthan gum [INCI:XANTHAN GUM] (ingredients of the phase C1).

On the other side, in a suitable vessel, Hydrolyte 5 2/016020 [INCI: PENTYLENE GLYCOL] and Microcare BNA [INCI: BENZYL ALCOHOL] were dissolved in purified water [INCI: WATER (AQUA)]. Then, Carbopol^{®} ultrez 10 [INCI:CARBOMER] was slowly added. In the next step, Arlatone MAP 160K [INCI: POTASSIUM CETYL PHOSPHATE] was added under stirring until its dispersion and the mixture of ingredients was heated to 70-75 °C. This set of ingredients constituted phase A.

The ingredients from phase B Finsolv-TN [INCI: C12-15 ALKYL BENZOATE], Phytocream 2000 [INCI: GLYCERYL STEARATE, CETEARYL ALCOHOL, POTASSIUM PALMITOYL HYDROLYZED WHEAT PROTEIN], Massocare EC [INCI: ETHYLHEXYL COCOATE], phenoxyethanol [INCI: PHENOXYETHANOL] and vitamin E acetate [INCI:TOCOPHERYL ACETATE] were also dissolved at 70-75°C. Once dissolved they were slowly added, under turbine stirring to the mixture of ingredients of the phase A.

The mixture was cooled to 50°C and Silicone dc 200 [INCI: DIMETHICONE] together with the components of phase C1 were added to the mixture of ingredients of A and B.

Under turbine stirring Sepigel 305 [INCI: WATER (AQUA), POLYACRYLAMIDE, C13-14 ISOPARAFFIN, LAURETH- 7] (phase D) was added to the emulsion resulting from the mixture of the different phases. Then, phase E, Perfume Ocean 12720 [INCI: FRAGRANCE (PARFUM)] was added to the mixture under turbine stirring. The pH was adjusted to 6.0-6.5 by dropwise addition under stirring of sodium hydroxide [INCI: SODIUM HYDROXIDE] (phase F).

The proportion of the obtained cosmetic composition are shown in table 6:

**Table 6**

| | INGREDIENT | % in weight |
|---|---|---|
| A | WATER (AQUA) | 75.00 |
| A | PENTYLENE GLYCOL | 5.00 |
| A | BENZYL ALCOHOL | 1.00 |
| A | CARBOMER | 0.50 |
| A | POTASSIUM CETYL PHOSPHATE | 0.50 |
| B | C12-15ALKYL BENZOATE | 5.00 |
| B | GLYCERYL STEARATE | 2.05 |
| B | CETEARYL ALCOHOL | 2.05 |
| B | POTASSIUM PALMITOYL HYDROLYZED WHEAT PROTEIN | 0.90 |
| B | ETHYLHEXYL COCOATE | 2.50 |
| B | PHENOXYETHANOL | 0.90 |
| B | TOCOPHERYL ACETATE | 0.50 |
| C | DIMETHICONE | 1.00 |
| C1 | WATER (AQUA) | 1.41 |
| C1 | Exopolysaccharide of the strain CNCM I-4239 | 0.02 |
| C1 | DISODIUM PHOSPHATE | 0.03 |
| C1 | SODIUM PHOSPHATE | 0.02 |
| C1 | XANTHAN GUM | 0.02 |
| C1 | PROPANEDIOL | 0.49 |
| C1 | GLYCERYL CAPRYLATE | 0.01 |
| D | WATER (AQUA) | 0.34 |
| D | POLYACRYLAMIDE | 0.40 |
| D | C13-14 ISOPARAFFIN | 0.20 |
| D | LAU RETH- 7 | 0.06 |
| E | FRAGRANCE (PARFUM) | 0.10 |
| F | SODIUM HYDROXIDE 20% | q.s. |

## Claims

1. Exopolysaccharide of the strain of *Vibrio sp.* with deposit number CNC I-4239, for use in the treatment and/or prevention of pain, inflammation, itching or hyperhidrosis of the skin and/or mucous membranes, wherein the exopolysaccharide comprises from 30 to 55 % by weight glucuronic acid, from 30 to 60 % by weight N-acetylglucosamine, and from 5 to 20 % by weight N-acetylgalactosamine.

2. Exopolysaccharide for use according to claim 1, wherein the exopolysaccharide comprises from 35 to 50 % by weight glucuronic acid, from 40 to 55 % by weight N-acetylglucosamine, and from 5 to 15 % by weight N-acetylgalactosamine.

3. Exopolysaccharide for use according to claim 1 or claim 2, wherein the exopolysaccharide has a chemical modification selected from the group formed by phosphorylation, sulfonation, acylation, esterification, formation of metallic complexes of the exopolysaccharide and/or chemical sulfation.

4. Exopolysaccharide for use according to any one of claims 1 to 3, wherein the treatment inhibits neuronal exocytosis.

5. Use of the exopolysaccharide of the strain of the species *Vibrio sp.* with deposit number CNCM I-4239 for the cosmetic, non-therapeutic treatment and/or care of the skin, mucous membranes, hair and/or nails selected from the treatment and/or prevention of expression wrinkles, treatment and/or prevention of perspiration, treatment and/or care of skin disorders selected from the group formed by warts, calluses, treatment stimulating hair growth and/or prevention of hair loss, wherein the exopolysaccharide comprises from 30 to 55 % by weight glucuronic acid, from 30 to 60 % by weight N-acetylglucosamine, and from 5 to 20 % by weight N-acetylgalactosamine.

6. Use of the exopolysaccharide according to claim 5, wherein the exopolysaccharide comprises from 35 to 50 % by weight glucuronic acid, from 40 to 55 % by weight N-acetylglucosamine, and from 5 to 15 % by weight N-acetylgalactosamine.

7. Use of the exopolysaccharide according to claim 5 or claim 6, wherein the exopolysaccharide has a chemical modification selected from the group formed by phosphorylation, sulfonation, acylation, esterification, formation of metallic complexes of the exopolysaccharide and/or chemical sulfation.

8. Use of the exopolysaccharide according to any one of claims 5 to 7, wherein the cosmetic, non-therapeutic treatment and/or care inhibits the neuronal exocytosis.

9. Cosmetic or dermopharmaceutical composition which comprises a cosmetically or dermopharmaceutically effective quantity of the exopolysaccharide of the strain Vibrio sp. with deposit number CNC I-4239, and at least one cosmetically and/or dermopharmaceutically acceptable excipient, adjuvant and/or ingredient, wherein the exopolysaccharide comprises from 30 to 55 % by weight glucuronic acid, from 30 to 60 % by weight N-acetylglucosamine, and from 5 to 20 % by weight N-acetylgalactosamine.

10. Cosmetic or dermopharmaceutical composition according to claim 9, wherein the exopolysaccharide comprises from 35 to 50 % by weight glucuronic acid, from 40 to 55 % by weight N-acetylglucosamine, and from 5 to 15 % by weight N-acetylgalactosamine.

11. Cosmetic or dermopharmaceutical composition according to claim 9 or claim 10, wherein the exopolysaccharide has a chemical modification selected from the group formed by phosphorylation, sulfonation, acylation, esterification, formation of metallic complexes of the exopolysaccharide and/or chemical sulfation.

12. Cosmetic or dermopharmaceutical composition according to any one of claims 9 to 11, wherein the exopolysaccharide is incorporated into a cosmetically or dermopharmaceutically acceptable delivery system or sustained release system selected from the group formed by liposomes, mixed liposomes, oleosomes, niosomes, ethosomes, milliparticles, microparticles, nanoparticles, solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules, nanocapsules, microemulsions and nanoemulsions.

13. Cosmetic or dermopharmaceutical composition, according to any of claims 9 to 12, wherein this composition is presented in a formulation selected from the group formed by creams, multiple emulsions, solutions, liquid crystals, anhydrous compositions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols.

14. Cosmetic or dermopharmaceutical composition, according to any of claims 9 to 13, wherein said excipient, adjuvant and/or ingredient is selected from the group formed by inhibitors of neuronal exocytosis, anticholinergic agents, muscle contraction inhibiting agents, antiaging agents, anti-wrinkle agents, antiperspirant agents, anti-inflammatory agents and/or analgesics, anti-itching agents, calming agents, anesthetic agents, agents inhibiting acetylcholine receptor clustering, agents that inhibit acetylcholinesterase, skin relaxant agents, melanin synthesis stimulating or inhibiting agents, whitening or depigmenting agents, propigmenting agents, self-tanning agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin conditioners, humectants, substances that retain moisture, alpha hydroxyacids, beta hydroxyacids, moisturizers, epidermal hydrolytic enzymes, vitamins, amino acids, proteins, pigments or colorants, dyes, biopolymers, gelling polymers, thickeners, surfactants, softening agents, emulsifiers, binding agents, preservatives, agents able to reduce or treat bags under the eyes, exfoliating agents, desquamating agents, keratolytic agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, collagen synthesis-stimulating agents, elastin synthesis-stimulating agents, decorin synthesis-stimulating agents, laminin synthesis-stimulating agents, defensin synthesis-stimulating agents, chaperone synthesis-stimulating agents, cAMP synthesis-stimulating agents, agents that modulate AQP-3, agents that modulate aquaporin synthesis, proteins from the aquaporin family, hyaluronic acid synthesis-stimulating agents, gycosaminoglycan synthesis-stimulating agents, fibronectin synthesis-stimulating agents, sirtuin synthesis-stimulating agents, sirtuin activating agents, heat shock proteins, heat shock protein synthesis-stimulating agents, agents stimulating the synthesis of lipids and components of the stratum corneum, ceramides, fatty acids, agents that inhibit collagen degradation, agents that inhibit matrix metalloproteinases, agents that inhibit elastin degradation, agents that inhibit serine proteases, agents stimulating fibroblast proliferation, agents stimulating keratinocyte proliferation, agents stimulating adipocyte proliferation, agents stimulating melanocyte proliferation, agents stimulating keratinocyte differentiation, agents that accelerate or delay adipocyte differentiation, antihyperkeratosis agents, comedolytic agents, anti-psoriasis agents, DNA repairing agents, DNA protecting agents, stem cell protecting agents, stabilizers, agents for the treatment and/or care of sensitive skin, firming agents, anti-stretch mark agents, binding agents, agents regulating sebum production, lipolytic agents or agents stimulating lipolysis, adipogenic agents, agents that modulate PGC-1α expression, agents that modulate PPARγ, agents that increase or reduce the triglyceride content of adipocytes, anti-cellulite agents, PAR-2 activity inhibiting agents, agents stimulating healing, coadjuvant healing agents, agents stimulating reepithelialization, coadjuvant reepithelialization agents, cytokines, growth factors, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, hair loss delaying agents, perfumes, cosmetic deodorant agents and/or body odor absorbent agent and/or body odor masking agent, chelating agents, plant extracts, essential oils, marine extracts, agents obtained from a biotechnological process, mineral salts, cell extracts, sunscreens and organic or mineral photoprotective agents active against ultraviolet A and/or B rays and/or infrared A rays, or mixtures thereof.

15. Exopolysaccharide for use according to any of claims 1 to 4, wherein the exopolysaccharide is present in a cosmetic or dermopharmaceutical composition defined in any one of claims 9 to 14.

16. Use according to any one of claims 5 to 8, wherein the exopolysaccharide is present in a cosmetic or dermopharmaceutical composition defined in any one of claims 9 to 14.

## Patentansprüche

1. Exopolysaccharid des Stammes von *Vibrio sp.* mit der Hinterlegungsnummer CNC 1-4239, zur Verwendung bei der Behandlung und/oder Prävention von Schmerz, Entzündung, Jucken oder Hyperhidrose der Haut und/oder der Schleimhäute, wobei das Exopolysaccharid Folgendes umfasst: 30 bis 55 Gewichts-% Glucuronsäure, 30 bis 60 Gewichts-% N-Acetylglucosamin und 5 bis 20 Gewichts-% N-Acetylgalactosamin.

2. Exopolysaccharid zur Verwendung nach Anspruch 1, wobei das Exopolysaccharid Folgendes umfasst: 35 bis 50 Gewichts-% Glucuronsäure, 40 bis 55 Gewichts-% N-Acetylglucosamin und 5 bis 15 Gewichts-% N-Acetylgalactosamin.

3. Exopolysaccharid zur Verwendung nach Anspruch 1 oder Anspruch 2, wobei das Exopolysaccharid eine chemische Modifikation aufweist, die aus der Gruppe ausgewählt wird, die aus Phosphorylierung, Sulfierung, Acylierung, Veresterung, Bildung von Metallkomplexen des Exopolysaccharids und/oder chemischer Sulfatierung besteht.

4. Exopolysaccharid zur Verwendung nach einem der Ansprüche 1 bis 3, wobei die Behandlung neuronale Exozytose hemmt.

5. Verwendung des Exopolysaccharids des Stammes der Spezies Vibrio sp. mit der Hinterlegungsnummer CNCM 1-4239 zur kosmetischen, nicht-therapeutischen Behandlung und/oder Pflege der Haut, Schleimhäute, Haare und/oder Nägel, ausgewählt aus der Behandlung und/oder Prävention von Mimikfalten, Behandlung und/oder Prävention von Perspiration, Behandlung und/oder Pflege von Hauterkrankungen, die aus der Gruppe ausgewählt werden, die aus Warzen, Schwielen, Behandlungsstimulierung des Haarwuchses und/oder Prävention von Haarausfall besteht, wobei das Exopolysaccharid Folgendes umfasst: 30 bis 55 Gewichts-% Glucuronsäure, 30 bis 60 Gewichts-% N-Acetylglucosamin und 5 bis 20 Gewichts-% N-Acetylgalactosamin.

6. Verwendung des Exopolysaccharids nach Anspruch 5, wobei das Exopolysaccharid Folgendes umfasst: 35 bis 50 Gewichts-% Glucuronsäure, 40 bis 55 Gewichts-% N-Acetylglucosamin und 5 bis 15 Gewichts-% N-Acetylgalactosamin.

7. Verwendung des Exopolysaccharids nach Anspruch 5 oder Anspruch 6, wobei das Exopolysaccharid eine chemische Modifikation aufweist, die aus der Gruppe ausgewählt wird, die aus Phosphorylierung, Sulfierung, Acylierung, Veresterung, Bildung von Metallkomplexen des Exopolysaccharids und/oder chemischer Sulfatierung besteht.

8. Verwendung des Exopolysaccharids nach einem der Ansprüche 5 bis 7, wobei die kosmetische, nicht-therapeutische Behandlung und/oder Pflege die neuronale Exozytose hemmt.

9. Kosmetische oder dermopharmazeutische Zusammensetzung, die eine kosmetisch oder dermopharmazeutisch wirksame Menge des Exopolysaccharids des Stammes Vibrio sp. mit der Hinterlegungsnummer CNC 1-4239 und mindestens einen kosmetisch und/oder dermopharmazeutisch akzeptablen Träger, Hilfsstoff und/oder Inhaltsstoff umfasst, wobei das Exopolysaccharid Folgendes umfasst: 30 bis 55 Gewichts-% Glucuronsäure, 30 bis 60 Gewichts-% N-Acetylglucosamin und 5 bis 20 Gewichts-% N-Acetylgalactosamin.

10. Kosmetische oder dermopharmazeutische Zusammensetzung nach Anspruch 9, wobei das Exopolysaccharid Folgendes umfasst: 35 bis 50 Gewichts-% Glucuronsäure, 40 bis 55 Gewichts-% N-Acetylglucosamin und 5 bis 15 Gewichts-% N-Acetylgalactosamin.

11. Kosmetische oder dermopharmazeutische Zusammensetzung nach Anspruch 9 oder Anspruch 10, wobei das Exopolysaccharid eine chemische Modifikation aufweist, die aus der Gruppe ausgewählt wird, die aus Phosphorylierung, Sulfierung, Acylierung, Veresterung, Bildung von Metallkomplexen des Exopolysaccharids und/oder chemischer Sulfatierung besteht.

12. Kosmetische oder dermopharmazeutische Zusammensetzung nach einem der Ansprüche 9 bis 11, wobei das Exopolysaccharid in einem kosmetisch oder dermopharmazeutisch akzeptablen Abgabesystem oder nachhaltigen Freigabesystem eingegliedert wird, das aus der Gruppe ausgewählt wird, die aus Folgendem besteht: Liposomen, gemischten Liposomen, Oleosomen, Niosomen, Ethosomen, Millimeterpartikeln, Mikropartikeln, Nanopartikeln, festen lipiden Nanopartikeln, nanostrukturierten Lipidträgern, Schwämmen, Cyclodextrinen, Bläschen, Micellen, gemischten Micellen von oberflächenaktiven Mitteln, gemischten Micellen mit oberflächenaktivem Phospholipid, Millimeterkügelchen, Mikrokügelchen und Nanokügelchen, Lipokügelchen, Millikapseln, Mikrokapseln, Nanokapseln, Mikroemulsionen und Nanoemulsionen.

13. Kosmetische oder dermopharmazeutische Zusammensetzung nach einem der Ansprüche 9 bis 12, wobei diese Zusammensetzung in einer Formulierung bereitgestellt wird, die aus der Gruppe ausgewählt wird, die aus Folgendem besteht: Cremes, multiplen Emulsionen, Lösungen, Flüssigkristallen, wasserfreien Zusammensetzungen, wasserfreien Zusammensetzungen, wässrigen Dispersionen, Ölen, Milchen, Balsamen, Schäumen, Lotionen, Gelen, Creme-Gelen, Wasser-Alkohol-Lösungen, Wasser-Glykol-Lösungen, Hydrogelen, Einreibemitteln, Seren, Seifen, Shampoos, Spülungen, Seren, Polysaccharidfilmen, Salben, Schaumfestigern, Pomaden, Pulvern, Stäben, Stiften und Sprays oder Aerosolen.

14. Kosmetische oder dermopharmazeutische Zusammensetzung nach einem der Ansprüche 9 bis 13, wobei der Träger, Hilfsstoff und/oder Inhaltsstoff aus der Gruppe ausgewählt werden, die aus Folgendem besteht: Inhibitoren der neuronalen Exozytose, Anticholinergika, Muskelkontraktion-hemmende Mittel, Alterungsschutzmittel, Antifaltenmittel, transpirationshemmende Mittel, entzündungshemmende Mittel und/oder Analgetika, Antijuckreizmittel, Beruhigungsmittel, Betäubungsmittel, Mittel zur Hemmung des Acetylcholin-Rezeptor-Clustering, Mittel zur Hemmung von Acetylcholinesterase, Hautberuhigungsmittel, Melaninsynthese-stimulierende oder -hemmende Mittel, Bleich- oder Depigmentierungsmittel, pigmentierungsfördernde Mittel, Selbstbräunungsmittel, NO-Synthase-hemmende Mittel, 5α-Reduktase-hemmende Mittel, Lysyl- und/oder Prolylhydroxylase-hemmende Mittel, Antioxidantien, Fänger von freien Radikalen und/oder Mittel gegen Luftverschmutzung, Fänger von reaktiven Carbonylspezies, Antiglykationsmittel, Antihistaminika, antivirale Mittel, Antiparasitika, Emulgatoren, Weichmacher, organische Lösungsmittel, Flüssigtreibstoffe, Hautkonditionierer, Feuchthaltemittel, feuchtigkeithaltende Substanzen, Alphahydroxysäuren, Betahydroxysäuren, Feuchthaltemittel, epidermale hydrolytische Enzyme, Vitamine, Aminosäuren, Proteine, Pigmente oder Färbemittel, Farbstoffe, Biopolymere, gelierende Polymere, Verdickungsmittel, oberflächenaktive Mittel, Weichmacher, Emulgatoren, Bindemittel, Konservierungsmittel, Mittel zur Verringerung oder Behandlung von Augensäcken, Peeling-Mittel, abschilfernde Mittel, Keratolytika, antimikrobielle Mittel, Antimykotika, Fungistatika, bakterizide Mittel, bakteriostatische Mittel, Mittel zur Stimulierung der Synthese von dermalen oder epidermalen Makromolekülen und/oder zur Hemmung oder Verhinderung von deren Abbau, Kollagensynthese-stimulierende Mittel, Elastinsynthese-stimulierende Mittel, Decorinsynthese-stimulierende Mittel, Lamininsynthese-stimulierende Mittel, Defensinsynthese-stimulierende Mittel, Chaperonsynthese-stimulierende Mittel, cAMP-Synthese-stimulierende Mittel, AQP-3-modulierende Mittel, Aquaporinsynthesemodulierende Mittel, Proteine aus der Aquaporinfamilie, Hyaluronsäuresynthesestimulierende Mittel, Gycosaminoglycansynthese-stimulierende Mittel, Fibronektinsynthese-stimulierende Mittel, Sirtuinsynthese-stimulierende Mittel, Sirtuinaktivierungsmittel, Hitzeschockproteine, Hitzeschock-Proteinsynthesestimulierende Mittel, Mittel, die die Synthese von Lipiden und Komponenten des Stratum corneum stimulieren, Ceramide, Fettsäuren, Kollagenabbau-hemmende Mittel, Matrix-Metalloproteinase-hemmende Mittel, Elastin-Abbau-hemmende Mittel, Serinproteasehemmende Mittel, Mittel, die die Proliferation von Fibroblasten stimulieren, Mittel, die die Proliferation von Keratinocyten stimulieren, Mittel, die die Proliferation von Adipozyten stimulieren, Mittel, die die Proliferation von Melanozyten stimulieren, Mittel, die die Keratinozytendifferenzierung stimulieren, Mittel, die die Adipocytendifferenzierung beschleunigen oder verzögern, Antihyperkeratosemittel, komedolytische Mittel, Antipsoriasismittel, DNA-Instandsetzungsmittel, DNA-Schutzmittel, Stammzellschutzmittel, Stabilisatoren, Mittel zur Behandlung und/oder Pflege von empfindlicher Haut, Festigungsmittel, Antidehnungsstreifenmittel, Bindemittel, Talgproduktion-regulierende Mittel, Iipolytische Mittel oder Lipolysestimulierende Mittel, adipogene Mittel, Mittel, die die PGC-1α-Expression modulieren, PPARγ-modulierende Mittel, Mittel, die den Triglycerid-Gehalt von Adipozyten erhöhen oder verringern, Antizellulitismittel, PAR-2-Aktivität-hemmende Mittel, heilungsstimulierende Mittel, Coadjuvansheilmittel, Reepithelialisierung-stimulierende Mittel, Coadjuvans-Reepithalisierungsmittel, Cytokine, Wachstumsfaktoren, Mittel, die auf die Kapillarzirkulation und/oder Mikrozirkulation wirken, Angiogenese-stimulierende Mittel, Mittel, die die vaskuläre Permeabilität hemmen, venentonisierende Mittel, Mittel, die auf den Zellstoffwechsel wirken, Mittel, die die dermale-epidermale Verbindung verbessern, Haarwachstumsmittel, Haarwachstum-hemmende oder verzögernde Mittel, Haarausfallverzögerungsmittel, Duftstoffe, kosmetische Deodorant-Wirkstoffe und/oder Körpergeruch-absorbierende Mittel und/oder Körpergeruch-Maskierungsmittel, Chelatbildner, Pflanzenextrakte, ätherische Öle, Meeresextrakte, Mittel, die aus einem biotechnologischen Verfahren gewonnen werden, Mineralsalze, Zellextrakte, Sonnenschutzmittel und organische oder mineralische Lichtschutzmittel, die gegen ultraviolette A- und/oder B-Strahlung und/oder Infrarot-A-Strahlung wirken, oder Mischungen davon.

15. Exopolysaccharid zur Verwendung nach einem der Ansprüche 1 bis 4, wobei das Exopolysaccharid in einer kosmetischen oder dermopharmazeutischen Zusammensetzung nach einem der Ansprüche 9 bis 14 vorliegt.

16. Verwendung nach einem der Ansprüche 5 bis 8, wobei das Exopolysaccharid in einer kosmetischen oder dermopharmazeutischen Zusammensetzung nach einem der Ansprüche 9 bis 14 vorliegt.

## Revendications

1. Exopolysaccharide de souche de *Vibrio sp.* sous le numéro de dépôt CNC 1-4239, à utiliser dans le traitement et/ou la prévention de la douleur, de l'inflammation, des démangeaisons ou de hyperhydrose de la peau et/ou des membranes muqueuses, où l'exopolysaccharide comprend de 30 à 55 % en poids d'acide glucuronique, de 30 à 60 % en poids de N-acétylglucosamine, et de 5 à 20 % en poids de N-acétylgalactosamine.

2. Exopolysaccharide à utiliser selon la revendication 1, où l'exopolysaccharide comprend de 35 à 50 % en poids d'acide glucuronique, de 40 à 55 % en poids de N-acétylglucosamine, et de 5 à 15 % en poids de N-acétylgalactosamine.

3. Exopolysaccharide à utiliser selon la revendication 1 ou la revendication 2, où l'exopolysaccharide présente une modification chimique sélectionnée dans le groupe formé par la phosphorylation, la sulfonation, l'acylation, l'estérification, la formation des complexes métalliques de l'exopolysaccharide et/ou la sulfatation chimique.

4. Exopolysaccharide à utiliser selon l'une quelconque des revendications 1 à 3, où le traitement inhibe l'exocytose neuronale.

5. Utilisation de l'exopolysaccharide de souche d'espèces de *Vibrio sp.* sous le numéro de dépôt CNCM 1-4239 pour le traitement cosmétique, non thérapeutique et/ou les soins de la peau, des membranes muqueuses, des cheveux et/ou des ongles sélectionnée parmi le traitement et/ou la prévention des rides d'expression, le traitement et/ou la prévention de la transpiration, le traitement et/ou les soins des troubles cutanés sélectionnés dans le groupe formé par les verrues, les cals, le traitement de stimulation de la croissance des cheveux et/ou la prévention de la perte de cheveux, où l'exopolysaccharide comprend de 30 à 55 % en poids d'acide glucuronique, de 30 à 60 % en poids de N-acétylglucosamine, et de 5 à 20 % en poids de N-acétylgalactosamine.

6. Utilisation de l'exopolysaccharide selon la revendication 5, où l'exopolysaccharide comprend de 35 à 50 % en poids d'acide glucuronique, de 40 à 55 % en poids de N-acétylglucosamine, et de 5 à 15 % en poids de N-acétylgalactosamine.

7. Utilisation de l'exopolysaccharide selon la revendication 5 ou la revendication 6, où l'exopolysaccharide présente une modification chimique sélectionnée dans le groupe formé par la phosphorylation, la sulfonation, l'acylation, l'estérification, la formation des complexes métalliques de l'exopolysaccharide et/ou la sulfatation chimique.

8. Utilisation de l'exopolysaccharide selon l'une quelconque des revendications 5 à 7, où le traitement cosmétique, non thérapeutique et/ou les soins inhibent l'exocytose neuronale.

9. Composition cosmétique ou dermopharmaceutique qui comprend une quantité cosmétiquement ou dermopharmaceutiquement efficace de l'exopolysaccharide de souche de *Vibrio sp.* sous le numéro de dépôt CNC 1-4239, et au moins un excipient, adjuvant et/ou ingrédient cosmétiquement et/ou dermopharmaceutiquement acceptables, où l'exopolysaccharide comprend de 30 à 55 % en poids d'acide glucuronique, de 30 à 60 % en poids de N-acétylglucosamine, et de 5 à 20 % en poids de N-acétylgalactosamine.

10. Composition cosmétique ou dermopharmaceutique selon la revendication 9, où l'exopolysaccharide comprend de 35 à 50 % en poids d'acide glucuronique, de 40 à 55 % en poids de N-acétylglucosamine, et de 5 à 15 % en poids de N-acétylgalactosamine.

11. Composition cosmétique ou dermopharmaceutique selon la revendication 9 ou la revendication 10, où l'exopolysaccharide présente une modification chimique sélectionnée dans le groupe formé par la phosphorylation, la sulfonation, l'acylation, l'estérification, la formation des complexes métalliques de l'exopolysaccharide et/ou la sulfatation chimique.

12. Composition cosmétique ou dermopharmaceutique selon l'une quelconque des revendications 9 à 11, où l'exopolysaccharide est incorporé dans un système d'administration cosmétiquement ou dermopharmaceutiquement acceptable ou un système à libération prolongée sélectionné dans le groupe formé par les liposomes, les liposomes mixtes, les oléosomes, les niosomes, les éthosomes, les milliparticules, les microparticules, les nanoparticules, les nanoparticules lipidiques solides, les supports lipides nanostructurés, les éponges, les cyclodextrines, les vésicules, les micelles, les micelles mixtes de tensioactifs, les micelles mixtes de tensioactif-phospholipide, les millisphères, les microsphères et les nanosphères, les liposphères, les millicapsules, les microcapsules, les nanocapsules, les microémulsions et les nanoémulsions.

13. Composition cosmétique ou dermopharmaceutique, selon l'une quelconque des revendications 9 à 12, où cette composition est présentée dans une formulation sélectionnée dans le groupe formé par les crèmes, les émulsions multiples, les solutions, les cristaux liquides, les compositions anhydres, les compositions anhydres, les dispersions aqueuses, les huiles, les laits, les baumes, les mousses, les lotions, les gels, les gels crèmes, les solutions hydroalcoholiques, les solutions hydroglycoliques, les hydrogels, les liniments, les sérums, les savons, les shampoings, les conditionneurs, les sérums, les films polysaccharidiques, les onguents, les mousses, les pommades, les poudres, les barres, les crayons et les pulvériseurs ou les aérosols.

14. Composition cosmétique ou dermopharmaceutique, selon l'une quelconque des revendications 9 à 13, où ledit excipient, adjuvant et/ou ingrédient est sélectionné dans le groupe formé par les inhibiteurs de l'exocytose neuronale, les agents anticholinergiques, les agents inhibant la contraction musclaire, les agents antivieillissement, les agents antirides, les agents antitranspirants, les agents anti-inflammatoires et/ou les analgésiques, les agents anti-démangeaisons, les agents calmants, les agents anesthésiques, les agents inhibant l'amas des récepteurs de l'acétylcholine, les agents qui inhibent l'acétylcholinestérase, les agents relaxants cutanés, les agents de stimulation ou d'inhibition de la synthèse de la mélanine, les agents blanchissants ou dépigmentants, les agents propigmentants, les agents auto-bronzants, les agents inhibant la NO-synthase, les agents inhibant la 5α-réductase, les agents inhibant la lysyl- et/ou prolyl hydroxylase, les agents antioxydants, les purificateurs de radicaux libres et/ou les agents anti-pollution atmosphérique, les purificateurs des espèces carbonyle réactives, les agents anti-glycation, les agents antihistaminiques, les agents antiviraux, les agents antiparasitaires, les émulsifiants, les émollients, les solvants organiques, les agents propulseurs de liquide, les conditionneurs cutanés, les agents humectants, les substances qui retiennent l'humidité, les alpha hydroxyacides, les bêta hydroxyacides, les agents hydratants, les enzymes hydrolytiques épidermiques, les vitamines, les acides aminés, les protéines, les pigments ou les colorants, les teintures, les biopolymères, les polymères gélifiants, les épaississants, les tensioactifs, les agents émollients, les émulsifiants, les agents liants, les conservateurs, les agents capables de réduire ou de traiter les poches sous les yeux, les agents exfoliants, les agents desquamants, les agents kératolytiques, les agents antimicrobiens, les agents antifongiques, les agents fongistatiques, les agents bactéricides, les agents bactériostatiques, les agents stimulant la synthèse des macromolécules dermiques ou épidermiques et/ou capables d'inhiber ou de prévenir leur dégradation, les agents de stimulation de la synthèse du collagène, les agents de stimulation de la synthèse de l'élastine, les agents de stimulation de la synthèse de la décorine, les agents de stimulation de la synthèse de la laminine, les agents de stimulation de la synthèse de la défensine, les agents de stimulation de la synthèse de la chaperone, les agents de stimulation de la synthèse de l'AMPc, les agents qui modulent l'AQP-3, les agents qui modulent la synthèse de l'aquaporine, les protéines de la famille de l'aquaporine, les agents de stimulation de la synthèse de l'acide hyaluronique, les agents de stimulation de la synthèse du glycosaminoglycane, les agents de stimulation de la synthèse de la fibronectine, les agents de stimulation de la synthèse de la sirtuine, les agents d'activation de la sirtuine, les protéines de choc thermique, les agents de stimulation de la synthèse des protéines de choc thermique, les agents de stimulation de la synthèse des lipides et des composants de la couche cornée de l'épiderme, les céramides, les acides gras, les agents qui inhibent la dégradation du collagène, les agents qui inhibent les métalloprotéinases de la matrice, les agents qui inhibent la dégradation de l'élastine, les agents qui inhibent les sérine protéases, les agents stimulant la prolifération des fibroblastes, les agents stimulant la prolifération des kératinocytes, les agents stimulant la prolifération des adipocytes, les agents stimulant la prolifération des mélanocytes, les agents stimulant la différenciation des kératinocytes, les agents qui accélèrent ou qui retardent la différenciation des adipocytes, les agents antihyperkératoses, les agents comédolytiques, les agents anti-psoriasis, les agents de réparation de l'ADN, les agents de protection de l'ADN, les agents de protection des cellules souches, les agents stabilisants, les agents de traitement et/ou de soins des peaux sensibles, les agents raffermissants, les agents anti-vergetures, les agents liants, les agents régulant la production de sébum, les agents lipolytiques ou les agents stimulant la lipolyse, les agents adipogéniques, les agents qui modulent l'expression de PGC-1α, les agents qui modulent PPARγ, les agents qui accroissent ou réduisent la teneur triglycéridique des adipocytes, les agents anti-cellulite, les agents d'inhibition de l'activité de PAR-2, les agents stimulant la guérison, les agents de guérison coadjuvants, les agents stimulant la réépithélialisation, les agents coadjuvants de réépithélialisation, les cytokines, les facteurs de croissance, les agents agissant sur la circulation et/ou la microcirculation capillaire, les agents stimulant l'angiogenèse, les agents qui inhibent la perméabilité vasculaire, les agents vénotoniques, les agents agissant sur le métabolisme des cellules, les agents d'amélioration de la jonction derme-épiderme, les agents induisant la croissance capillaire, les agents inhibant ou retardant la croissance capillaire, les agents retardant la perte des cheveux, les parfums, les agents déodorants cosmétiques et/ou agent d'absorption des odeurs corporelles et/ou les agents de masquage des odeurs corporelles, les agents chélatants, les extraits végétaux, les huiles essentielles, les extraits marins, les agents obtenus par un procédé biotechnologique, les sels minéraux, les extraits de cellules, les filtres solaires et les agents photoprotecteurs organiques ou minéraux actifs contre les rayons ultraviolets A et/ou B et/ou les rayons infrarouges A, ou leurs mélanges.

15. Exopolysaccharide à utiliser selon l'une quelconque des revendications 1 à 4, où l'exopolysaccharide est présent dans une composition cosmétique ou dermopharmaceutique définie selon l'une quelconque des revendications 9 à 14.

16. Utilisation selon l'une quelconque des revendications 5 à 8, où l'exopolysaccharide est présent dans une composition cosmétique ou dermopharmaceutique définie selon l'une quelconque des revendications 9 à 14.
